# EUROPEAN PATENT SPECIFICATION

(11) **EP 4 121 523 B1**
(45) Date of publication and mention of the grant of the patent: **07.05.2025**
(21) Application number: 21772292.5
(22) Date of filing: 19.03.2021
(51) Int. Cl.: C12N 9/12, C12N 15/10, C12Q 1/6806, C12Q 1/6869

(54) **SINGLE CELL WORKFLOW FOR WHOLE GENOME AMPLIFICATION**
EINZELZELLENARBEITSABLAUF FÜR GESAMTGENOMAMPLIFIKATION
FLUX DE TRAVAIL UNICELLULAIRE POUR L'AMPLIFICATION DU GÉNOME ENTIER

(30) Priority: 20.03.2020 US 202062992772 P
(43) Date of publication of application: 25.01.2023
(73) Proprietor: Mission Bio, Inc., South San Francisco, California 94080 (US)
(72) Inventor: DHINGRA, Dalia, South San Francisco, CA 94080 (US); RUFF, David, South San Francisco, CA 94080 (US)
(74) Representative: Elkington and Fife LLP
(86) International application number: PCT/US2021/023145
(87) International publication number: WO 2021/188889

(56) References cited:
- WO-A1-2018/119301
- WO-A1-2018/218226
- WO-A1-2018/226708
- WO-A1-2019/204229
- WO-A1-2019/222657
- WO-A1-2019/222688
- WO-A2-2019/033062
- US-A1- 2018 023 119

## Description

### GOVERNMENT RIGHTS

This invention was made with government support under Grant numbers: IAPRA-BAA-17-07,FELIX, awarded by the Intelligence Advanced Research Projects Activity (IARPA). The government has certain rights in the invention.

### BACKGROUND

Whole genome analysis of single cells remains elusive as conventional methods often result in sub-optimal sequencing coverage and/or low library complexity. For example, conventional methods result in high numbers of duplicate reads and low percentages of mapped reads. This prevents efficient analysis of portions of the whole genome. Sub-optimal sequencing coverage could miss the detection of one or more mutations present across the whole genome. Additionally, many conventional methods are low-throughput methods involving performing whole genome amplification on cells in plates. Thus, there is a need for a high-throughput, whole genome analysis of individual cells that achieves improved sequencing coverage and appropriate library complexity.

WO 2018/226708 A1 describes methods for preparing sequencing libraries for determining the methylation status of nucleic acids from a plurality of single cells, combining split-and-pool combinatorial indexing and bisulfite treatment techniques to characterize the methylation profiles of large numbers of single cells. WO 2019/204229 A1 discusses systems, methods, and compositions relating to hollow beads encapsulating single cells. These include performing multiple co-assays on a single cell encapsulated within a hollow bead, including nucleic acid sequencing, preparing nucleic acid libraries, determining methylation status, identifying genomic variants, or protein analysis. WO 2018/119301 A1 describes single cell genomic sequencing methods that include encapsulating single cells in molten gel droplets to facilitate bulk cell lysis and purification of genomic DNA in microgels.

### SUMMARY

The disclosure generally relates to methods and apparati for performing whole genome amplification in a single-cell workflow analysis. The disclosed single cell workflow analysis achieves sequencing coverage over the whole genome (e.g., whole human genome including 22 pairs of autosomal chromosomes and 1 pair of sex chromosomes). Generally, the single cell workflow involves encapsulating and lysing individual cells within droplets. Genomic DNA (gDNA) is released from chromatin using a protease. The released genomic DNA undergoes tagmentation, which involving cleaving and inserting adaptor sequences into the genomic DNA. In various embodiments, tagmentation occurs simultaneously as the genomic DNA is released. In various embodiments, tagmentation occurs prior to release of the genomic DNA. The tagmented DNA is extended to fill in any gaps resulting from the insertion of the adaptor sequences. In various embodiments, tagmentation of the genomic DNA occurs within the same droplet in which the genomic DNA was released from chromatin. In various embodiments, tagmentation occurs in a second droplet. The tagmented DNA further undergoes nucleic acid amplification. The resulting amplicons are sequenced for generating a whole genome sequencing library.

Disclosed herein is a method for performing whole genome sequencing, the method comprising: providing a cell and reagents within a first droplet, the reagents comprising a lysing reagent and a protease; lysing the cell using the lysing reagent within the first droplet; releasing genomic DNA using the protease within the first droplet by exposing the first droplet to a temperature between 30°C and 60°C; tagmenting, in either the first droplet or a second droplet, the released genomic DNA by: using a transposase at a temperature between 35°C and 55°C, cleaving the released genomic and incorporating adaptor sequences into the released genomic DNA; and filling in one or more gaps in the released genomic DNA created by incorporating the adaptor sequences at a temperature between 40°C and 100°C; and amplifying, in the second droplet, the tagmented genomic DNA to generate whole genome amplicons.

Additionally disclosed herein is a method for performing whole genome sequencing, the method comprising: encapsulating a cell and reagents within a first droplet, the reagents comprising a lysing reagent and a protease; lysing the cell using the lysing reagent within the first droplet; releasing genomic DNA using the protease within the first droplet; encapsulating the released genomic DNA and a reaction mixture in a second droplet, the reaction mixture comprising a transposase and a DNA polymerase; tagmenting the released genomic DNA in the second droplet by: using the transposase, cleaving the released genomic and incorporating adaptor sequences into the released genomic DNA; using the DNA polymerase, filling in one or more gaps in the released genomic DNA created by incorporating the adaptor sequences; and amplifying, in the second droplet, the tagmented genomic DNA to generate whole genome amplicons.

Additionally disclosed herein is a method for performing whole genome sequencing, the method comprising: encapsulating a cell and reagents within a first droplet, the reagents comprising a lysing reagent, a protease, and either a reverse transcriptase or DNA polymerase; lysing the cell using the lysing reagent within the first droplet; releasing genomic DNA using the protease within the first droplet; tagmenting the released genomic DNA in the first droplet by: using the transposase, cleaving the released genomic and incorporating adaptor sequences into the released genomic DNA; using either the reverse transcriptase or DNA polymerase, filling in one or more gaps in the released genomic DNA created by incorporating the adaptor sequences; encapsulating the tagmented genomic DNA and a reaction mixture in a second droplet; and amplifying, in the second droplet, the tagmented genomic DNA using the reaction mixture to generate whole genome amplicons.

In various embodiments, tagmenting the released genomic DNA occurs within the first droplet. In various embodiments, tagmenting the released genomic DNA occurs within the second droplet. In various embodiments, the transposase is a MuA transposase or a Tn5 transposase. In various embodiments, the transposase is a pA-Tn5 fusion transposase. In various embodiments, the transposase is appended to the adaptor sequences.

In various embodiments, filling in one or more gaps in the released genomic DNA created by incorporating the adaptor sequences comprises using either a reverse transcriptase or DNA polymerase to fill in the one or more gaps. In various embodiments, the DNA polymerase is a HotStart DNA polymerase. In various embodiments, the DNA polymerase is a *Bacillus stearothermophilus* (Bst) DNA polymerase. In various embodiments, filling in one or more gaps in the released genomic DNA created by incorporating the adaptor sequences comprises exposing the released genomic DNA to an elevated temperature.

In various embodiments, the elevated temperature is at least 40°C. In various embodiments, the elevated temperature is at least 50°C. In various embodiments, the elevated temperature is at least 60°C. In various embodiments, filling in one or more gaps uses a reverse transcriptase, and wherein the elevated temperature is between 40°C and 50°C. In various embodiments, filling in one or more gaps uses a DNA polymerase, and wherein the elevated temperature is between 50°C and 70°C. In various embodiments, the released genomic DNA is exposed to the elevated temperature for between 3 and 8 minutes.

In various embodiments, methods disclosed herein further comprise exposing the released genomic DNA to a further elevated temperature. In various embodiments, the further elevated temperature is at least 70°C. In various embodiments, the further elevated temperature is between 70°C and 80°C. In various embodiments, the further elevated temperature is about 72°C. In various embodiments, the further elevated temperature is at least 75°C. In various embodiments, the released genomic DNA is exposed to the further elevated temperature for between 1 minute and 20 minutes. In various embodiments, the released genomic DNA is exposed to the further elevated temperature for about 10 minutes. In various embodiments, the released genomic DNA is exposed to the further elevated temperature for between 40 minutes and 80 minutes. In various embodiments, the released genomic DNA is exposed to the further elevated temperature for about 60 minutes.

In various embodiments, methods disclosed herein further comprise exposing the released genomic DNA to a yet further elevated temperature. In various embodiments, the yet further elevated temperature is between 90°C and 100°C. In various embodiments, the yet further elevated temperature is about 95°C. In various embodiments, the released genomic DNA is exposed to the yet further elevated temperature for between 1 minute and 40 minutes. In various embodiments, the released genomic DNA is exposed to the yet further elevated temperature for about 20 minutes. In various embodiments, releasing genomic DNA using the protease within the first droplet comprises exposing the first droplet to a temperature between 35°C and 55°C. In various embodiments, releasing genomic DNA using the protease within the first droplet comprises exposing the first droplet to a temperature of about 50°C.

In various embodiments, releasing the genomic DNA and cleaving the released genomic and incorporating adaptor sequences occur in parallel. In various embodiments, releasing the genomic DNA and cleaving the released genomic and incorporating adaptor sequences comprise: exposing the first droplet to a first temperature between 35°C and 55°C for between 20 minutes and 80 minutes; and exposing the first droplet to a second temperature between 45°C and 70°C for between 1 minute and 10 minutes.

In various embodiments, releasing the genomic DNA and cleaving the released genomic and incorporating adaptor sequences comprise: exposing the first droplet to a first temperature of about 37°C for about 30 minutes; and exposing the first droplet to a second temperature of about 65°C for about 5 minutes. In various embodiments, amplifying the tagmented genomic DNA occurs subsequent to tagmenting the released genomic DNA. In various embodiments, amplifying the tagmented genomic DNA comprises performing one or more cycles of denaturation, annealing, and nucleic acid extension. In various embodiments, amplifying the tagmented genomic DNA comprises performing an isothermal nucleic acid amplification reaction.

In various embodiments, the lysis reagent is NP40. In various embodiments, the lysis agent is 10% NP40. In various embodiments, the protease is proteinase K. In various embodiments, amplifying the tagmented genomic DNA using the reaction mixture to generate whole genome amplicons comprises incorporating cell barcodes into the whole genome amplicons. In various embodiments, methods disclosed herein further comprise sequencing the whole genome amplicons. In various embodiments, methods disclosed herein further comprise generating a whole genome sequencing library using the sequenced whole genome amplicons.

In various embodiments, at least 20% of sequence reads of the whole genome sequencing library are mapped. In various embodiments, at least 50% of sequence reads of the whole genome sequencing library are mapped. In various embodiments, at least 80% of sequence reads of the whole genome sequencing library are mapped. In various embodiments, at least 10% of sequence reads of the whole genome sequencing library have a correct structure. In various embodiments, at least 50% of sequence reads of the whole genome sequencing library have a correct structure. In various embodiments, at least 80% of sequence reads of the whole genome sequencing library have a correct structure. In various embodiments, less than 40% of sequence reads of the whole genome sequencing library are duplicated. In various embodiments, less than 10% of sequence reads of the whole genome sequencing library are duplicated.

Additionally disclosed herein is a system for performing whole genome sequencing, the system comprising: a device configured to perform steps comprising: providing a cell and reagents within a first droplet, the reagents comprising a lysing reagent and a protease; lysing the cell using the lysing reagent within the first droplet; releasing genomic DNA using the protease within the first droplet by exposing the first droplet to a temperature between 30°C and 60°C; tagmenting, in either the first droplet or a second droplet, the released genomic DNA by: using a transposase at a temperature between 35°C and 55°C, cleaving the released genomic and incorporating adaptor sequences into the released genomic DNA; and filling in one or more gaps in the released genomic DNA created by incorporating the adaptor sequences at a temperature between 40°C and 100°C; and amplifying, in the second droplet, the tagmented genomic DNA to generate whole genome amplicons.

Additionally disclosed herein is a system for performing whole genome sequencing, the system comprising: a device configured to perform steps comprising:
encapsulating a cell and reagents within a first droplet, the reagents comprising a lysing reagent and a protease; lysing the cell using the lysing reagent within the first droplet;
releasing genomic DNA using the protease within the first droplet; encapsulating the released genomic DNA and a reaction mixture in a second droplet, the reaction mixture comprising a transposase and a DNA polymerase; tagmenting the released genomic DNA in the second droplet by: using the transposase, cleaving the released genomic and incorporating adaptor sequences into the released genomic DNA; using the DNA polymerase, filling in one or more gaps in the released genomic DNA created by incorporating the adaptor sequences;
amplifying, in the second droplet, the tagmented genomic DNA to generate whole genome amplicons.

Additionally disclosed herein is a system for performing whole genome sequencing, the system comprising: a device configured to perform steps comprising: encapsulating a cell and reagents within a first droplet, the reagents comprising a lysing reagent, a protease, and either a reverse transcriptase or DNA polymerase; lysing the cell using the lysing reagent within the first droplet; releasing genomic DNA using the protease within the first droplet; tagmenting the released genomic DNA in the first droplet by: using the transposase, cleaving the released genomic and incorporating adaptor sequences into the released genomic DNA; using either the reverse transcriptase or DNA polymerase, filling in one or more gaps in the released genomic DNA created by incorporating the adaptor sequences; encapsulating the tagmented genomic DNA and a reaction mixture in a second droplet; and amplifying, in the second droplet, the tagmented genomic DNA using the reaction mixture to generate whole genome amplicons.

In various embodiments tagmenting the released genomic DNA occurs within the first droplet. In various embodiments tagmenting the released genomic DNA occurs within the second droplet. In various embodiments the transposase is a MuA transposase or a Tn5 transposase. In various embodiments, the transposase is a pA-Tn5 fusion transposase. In various embodiments, the transposase is appended to the adaptor sequences.

In various embodiments, filling in one or more gaps in the released genomic DNA created by incorporating the adaptor sequences comprises using either a reverse transcriptase or DNA polymerase to fill in the one or more gaps. In various embodiments, the DNA polymerase is a HotStart DNA polymerase. In various embodiments, the DNA polymerase is a *Bacillus stearothermophilus* (Bst) DNA polymerase. In various embodiments, filling in one or more gaps in the released genomic DNA created by incorporating the adaptor sequences comprises exposing the released genomic DNA to an elevated temperature. In various embodiments, the elevated temperature is at least 40°C. In various embodiments, the elevated temperature is at least 50°C. In various embodiments, the elevated temperature is at least 60°C. In various embodiments, filling in one or more gaps uses a reverse transcriptase, and wherein the elevated temperature is between 40°C and 50°C. In various embodiments, filling in one or more gaps uses a DNA polymerase, and wherein the elevated temperature is between 50°C and 70°C. In various embodiments, the released genomic DNA is exposed to the elevated temperature for between 3 and 8 minutes. In various embodiments, the device is configured to perform steps further comprising exposing the released genomic DNA to a further elevated temperature. In various embodiments, the further elevated temperature is at least 70°C. In various embodiments, the further elevated temperature is between 70°C and 80°C. In various embodiments, the further elevated temperature is about 72°C. In various embodiments, the further elevated temperature is at least 75°C. In various embodiments, the released genomic DNA is exposed to the further elevated temperature for between 1 minute and 20 minutes. In various embodiments, the released genomic DNA is exposed to the further elevated temperature for about 10 minutes. In various embodiments, the released genomic DNA is exposed to the further elevated temperature for between 40 minutes and 80 minutes. In various embodiments, the released genomic DNA is exposed to the further elevated temperature for about 60 minutes.

In various embodiments, the device if configured to perform steps further comprising exposing the released genomic DNA to a yet further elevated temperature. In various embodiments, the yet further elevated temperature is between 90°C and 100°C. In various embodiments, the yet further elevated temperature is about 95°C. In various embodiments, the released genomic DNA is exposed to the yet further elevated temperature for between 1 minute and 40 minutes. In various embodiments, the released genomic DNA is exposed to the yet further elevated temperature for about 20 minutes. In various embodiments, releasing genomic DNA using the protease within the first droplet comprises exposing the first droplet to a temperature between 35°C and 55°C. In various embodiments, releasing genomic DNA using the protease within the first droplet comprises exposing the first droplet to a temperature of about 50°C. In various embodiments, releasing the genomic DNA and cleaving the released genomic and incorporating adaptor sequences occur in parallel. In various embodiments, releasing the genomic DNA and cleaving the released genomic and incorporating adaptor sequences comprise: exposing the first droplet to a first temperature between 35°C and 55°C for between 20 minutes and 80 minutes; and exposing the first droplet to a second temperature between 45°C and 70°C for between 1 minute and 10 minutes.

In various embodiments, releasing the genomic DNA and cleaving the released genomic and incorporating adaptor sequences comprise: exposing the first droplet to a first temperature of about 37°C for about 30 minutes; and exposing the first droplet to a second temperature of about 65°C for about 5 minutes. In various embodiments, amplifying the tagmented genomic DNA occurs subsequent to tagmenting the released genomic DNA. In various embodiments, amplifying the tagmented genomic DNA comprises performing one or more cycles of denaturation, annealing, and nucleic acid extension. In various embodiments, amplifying the tagmented genomic DNA comprises performing an isothermal nucleic acid amplification reaction. In various embodiments, the lysis reagent is NP40. In various embodiments, the lysis agent is 10% NP40. In various embodiments, the protease is proteinase K. In various embodiments, amplifying the tagmented genomic DNA using the reaction mixture to generate whole genome amplicons comprises incorporating cell barcodes into the whole genome amplicons. In various embodiments, the device is further configured to perform steps further comprising sequencing the whole genome amplicons. In various embodiments, the device is further configured to perform steps further comprising generating a whole genome sequencing library using the sequenced whole genome amplicons.

In various embodiments, at least 20% of sequence reads of the whole genome sequencing library are mapped. In various embodiments, at least 50% of sequence reads of the whole genome sequencing library are mapped. In various embodiments, at least 80% of sequence reads of the whole genome sequencing library are mapped. In various embodiments, at least 10% of sequence reads of the whole genome sequencing library have a correct structure. In various embodiments, at least 50% of sequence reads of the whole genome sequencing library have a correct structure. In various embodiments, at least 80% of sequence reads of the whole genome sequencing library have a correct structure. In various embodiments, less than 40% of sequence reads of the whole genome sequencing library are duplicated. In various embodiments, less than 10% of sequence reads of the whole genome sequencing library are duplicated.

Additionally disclosed herein is a whole genome sequencing library comprising a plurality of sequence reads derived from genomic DNA across each chromosome of a single human cell, wherein at least 20% of the plurality of sequence reads of the whole genome sequencing library are mapped. In various embodiments, at least 50% of the plurality of sequence reads of the whole genome sequencing library are mapped. In various embodiments, at least 80% of the plurality of sequence reads of the whole genome sequencing library are mapped.

Additionally disclosed herein is a whole genome sequencing library comprising a plurality of sequence reads derived from genomic DNA across each chromosome of a single human cell, wherein at least 10% of sequence reads of the whole genome sequencing library have a correct structure. In various embodiments, at least 50% of the plurality of sequence reads of the whole genome sequencing library have a correct structure. In various embodiments, at least 80% of the plurality of sequence reads of the whole genome sequencing library have a correct structure.

Additionally disclosed herein is a whole genome sequencing library comprising a plurality of sequence reads derived from genomic DNA across each chromosome of a single human cell, wherein less than 40% of the plurality of sequence reads are duplicated. In various embodiments, less than 10% of the plurality of sequence reads are duplicated.

### BRIEF DESCRIPTION OF THE SEVERAL VIEWS OF THE DRAWINGS

These and other features, aspects, and advantages of the present invention will become better understood with regard to the following description and accompanying drawings. It is noted that wherever practicable similar or like reference numbers may be used in the figures and may indicate similar or like functionality. For example, a letter after a reference numeral, such as "adaptor sequence 420A," indicates that the text refers specifically to the element having that particular reference numeral. A reference numeral in the text without a following letter, such as "adaptor sequence 420," refers to any or all of the elements in the figures bearing that reference numeral (e.g. "adaptor sequence 420" in the text refers to reference numerals "adaptor sequence 420A," "adaptor sequence 420B," "adaptor sequence 420C," and/or "adaptor sequence 420D" in the figures).
Figure (FIG.) 1A shows an overall system environment for analyzing cell(s) through a single cell workflow analysis, in accordance with an embodiment.
FIG. 1B depicts a single cell workflow analysis to generate amplified nucleic acid molecules for sequencing, in accordance with an embodiment.
FIG. 2 is a flow process for analyzing nucleic acid sequences derived from analytes of the single cell, in accordance with an embodiment.
FIGs. 3A-3C depict the processing and releasing of analytes of a single cell in a droplet, in accordance with an embodiment in which tagmentation is performed in a second droplet.
FIGs. 3D-3G depict the processing and releasing of analytes of a single cell in a droplet, in accordance with an embodiment in which tagmentation is performed in a first droplet.
FIG. 4A depicts the tagmentation of genomic DNA, in accordance with an embodiment.
FIG. 4B depicts the amplification and barcoding of tagmented genomic DNA, in accordance with the embodiment shown in FIG. 4A.
FIG. 5 depicts an example computing device for implementing system and methods described in reference to FIGs. 1-4B.
FIG. 6A depicts a 10x microscopy image of droplets post-tagmentation and amplification according to a first experimental run in which tagmentation is performed in the second droplet.
FIG. 6B depicts normalized coverage across the whole genome according to a first experimental run in which tagmentation is performed in the second droplet.
FIG. 7A depicts a 10x microscopy image of droplets post-tagmentation and amplification according to a second experimental run in which tagmentation is performed in the second droplet.
FIG. 7B depicts normalized coverage across the whole genome according to a second experimental run in which tagmentation is performed in the second droplet.
FIG. 8A depicts a 10x microscopy image of droplets post-tagmentation and amplification according to a third experimental run in which tagmentation is performed in the second droplet.
FIG. 8B depicts normalized coverage across the whole genome according to a third experimental run in which tagmentation is performed in the second droplet.
FIG. 9 depicts whole genome library products generated across the first, second, and third experimental runs in which tagmentation is performed in the second droplet.
FIG. 10A and FIG. 10B depict normalized coverage across the whole genome of murine and human cell lines according to a fourth experimental run in which tagmentation is performed in the second droplet.
FIG. 11A and FIG. 11B depict normalized coverage across the whole genome of murine and human cell lines according to a fifth experimental run in which tagmentation is performed in the second droplet.
FIG. 12A and FIG. 12B depict normalized coverage across the whole genome of murine and human cell lines according to a sixth experimental run in which tagmentation is performed in the first droplet.
FIG. 13A and FIG. 13B depict normalized coverage across the whole genome of murine and human cell lines according to a seventh experimental run in which tagmentation is performed in the first droplet.
FIG. 14A and FIG. 14B depict normalized coverage across the whole genome of murine and human cell lines according to an eighth experimental run in which tagmentation is performed in the first droplet.
FIG. 15A and FIG. 15B depict library metrics across the sixth, seventh, and eighth experimental runs in which tagmentation is performed in the first droplet.

### DETAILED DESCRIPTION

### Definitions

Terms used in the claims and specification are defined as set forth below unless otherwise specified.

As used herein, the term "about" refers to a value that is within 10% above or below the value being described. For example, the term "about 5°C" indicates a range of from 4.5°C to 5.5°C.

The term "subject" or "patient" are used interchangeably and encompass an organism, human or non-human, mammal or non-mammal, male or female.

The term "sample" or "test sample" can include a single cell or multiple cells or fragments of cells or an aliquot of body fluid, such as a blood sample, taken from a subject, by means including venipuncture, excretion, ejaculation, massage, biopsy, needle aspirate, lavage sample, scraping, surgical incision, or intervention or other means known in the art.

The term "analyte" refers to a component of a cell. Cell analytes can be informative for characterizing a cell. Therefore, performing single-cell analysis of one or more analytes of a cell using the systems and methods described herein are informative for determining a state or behavior of a cell. Examples of an analyte include a nucleic acid (e.g., RNA, DNA, cDNA), a protein, a peptide, an antibody, an antibody fragment, a polysaccharide, a sugar, a lipid, a small molecule, or combinations thereof. In particular embodiments, a single-cell analysis involves analyzing two different analytes such as RNA and DNA. In such embodiments, the single-cell analysis is useful for whole genome and transcriptome analysis. In particular embodiments, a single-cell analysis involves analyzing three or more different analytes of a cell, such as RNA, DNA, and protein.

In some embodiments, the discrete entities as described herein are droplets. The terms "emulsion," "drop," "droplet," and "microdroplet" are used interchangeably herein, to refer to small, generally spherically structures, containing at least a first fluid phase, e.g., an aqueous phase (e.g., water), bounded by a second fluid phase (e.g., oil) which is immiscible with the first fluid phase. In some embodiments, droplets according to the present disclosure may contain a first fluid phase, e.g., oil, bounded by a second immiscible fluid phase, e.g. an aqueous phase fluid (e.g., water). In some embodiments, the second fluid phase will be an immiscible phase carrier fluid. Thus droplets according to the present disclosure may be provided as aqueous-in-oil emulsions or oil-in-aqueous emulsions. Droplets may be sized and/or shaped as described herein for discrete entities. For example, droplets according to the present disclosure generally range from 1 µm to 1000 µm, inclusive, in diameter. Droplets according to the present disclosure may be used to encapsulate cells, nucleic acids (e.g., DNA), enzymes, reagents, reaction mixture, and a variety of other components. The term emulsion may be used to refer to an emulsion produced in, on, or by a microfluidic device and/or flowed from or applied by a microfluidic device.

"Complementarity" or "complementary" refers to the ability of a nucleic acid to form hydrogen bond(s) or hybridize with another nucleic acid sequence by either traditional Watson-Crick or other non-traditional types. As used herein "hybridization," refers to the binding, duplexing, or hybridizing of a molecule only to a particular nucleotide sequence under low, medium, or highly stringent conditions, including when that sequence is present in a complex mixture (e.g., total cellular) DNA or RNA. *See, e.g.,* Ausubel, et al., Current Protocols In Molecular Biology, John Wiley & Sons, New York, N.Y., 1993. If a nucleotide at a certain position of a polynucleotide is capable of forming a Watson-Crick pairing with a nucleotide at the same position in an antiparallel DNA or RNA strand, then the polynucleotide and the DNA or RNAmolecule are complementary to each other at that position. The polynucleotide and the DNA or RNA molecule are "substantially complementary" to each other when a sufficient number of corresponding positions in each molecule are occupied by nucleotides that can hybridize or anneal with each other in order to affect the desired process. A complementary sequence is a sequence capable of annealing under stringent conditions to provide a 3'-terminal serving as the origin of synthesis of complementary chain.

The terms "amplify," "amplifying," "amplification reaction" and their variants, refer generally to any action or process whereby at least a portion of a nucleic acid molecule (referred to as a template nucleic acid molecule) is replicated or copied into at least one additional nucleic acid molecule. The additional nucleic acid molecule optionally includes sequence that is substantially identical or substantially complementary to at least some portion of the template nucleic acid molecule. The template nucleic acid molecule can be single-stranded or double-stranded and the additional nucleic acid molecule can independently be single-stranded or double-stranded. In some embodiments, amplification includes a template-dependent in vitro enzyme-catalyzed reaction for the production of at least one copy of at least some portion of the nucleic acid molecule or the production of at least one copy of a nucleic acid sequence that is complementary to at least some portion of the nucleic acid molecule. Amplification optionally includes linear or exponential replication of a nucleic acid molecule. In some embodiments, such amplification is performed using isothermal conditions; in other embodiments, such amplification can include thermocycling. In some embodiments, the amplification is a multiplex amplification that includes the simultaneous amplification of a plurality of target sequences in a single amplification reaction. At least some of the target sequences can be situated, on the same nucleic acid molecule or on different target nucleic acid molecules included in the single amplification reaction. In some embodiments, "amplification" includes amplification of at least some portion of DNA- and RNA-based nucleic acids alone, or in combination. The amplification reaction can include single or double-stranded nucleic acid substrates and can further include any of the amplification processes known to one of ordinary skill in the art. In some embodiments, the amplification reaction includes polymerase chain reaction (PCR). In some embodiments, the amplification reaction includes an isothermal amplification reaction such as LAMP. In the present invention, the terms "synthesis" and "amplification" of nucleic acid are used. The synthesis of nucleic acid in the present invention means the elongation or extension of nucleic acid from an oligonucleotide serving as the origin of synthesis. If not only this synthesis but also the formation of other nucleic acid and the elongation or extension reaction of this formed nucleic acid occur continuously, a series of these reactions is comprehensively called amplification. The polynucleic acid produced by the amplification technology employed is generically referred to as an "amplicon" or "amplification product."

Any nucleic acid amplification method may be utilized, such as a PCR-based assay, e.g., quantitative PCR (qPCR), or an isothermal amplification may be used to detect the presence of certain nucleic acids, e.g., genes of interest, present in discrete entities or one or more components thereof, e.g., cells encapsulated therein. Such assays can be applied to discrete entities within a microfluidic device or a portion thereof or any other suitable location. The conditions of such amplification or PCR-based assays may include detecting nucleic acid amplification over time and may vary in one or more ways.

A number of nucleic acid polymerases can be used in the amplification reactions utilized in certain embodiments provided herein, including any enzyme that can catalyze the polymerization of nucleotides (including analogs thereof) into a nucleic acid strand. Such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also includes fusion proteins comprising at least two portions linked to each other, where the first portion comprises a peptide that can catalyze the polymerization of nucleotides into a nucleic acid strand and is linked to a second portion that comprises a second polypeptide. In some embodiments, the second polypeptide can include a reporter enzyme or a processivity-enhancing domain. Optionally, the polymerase can possess 5' exonuclease activity or terminal transferase activity. In some embodiments, the polymerase can be optionally reactivated, for example through the use of heat, chemicals or re-addition of new amounts of polymerase into a reaction mixture. In some embodiments, the polymerase can include a hot-start polymerase or an aptamer-based polymerase that optionally can be reactivated.

"Forward primer binding site" and "reverse primer binding site" refer to the regions on the template nucleic acid and/or the amplicon to which the forward and reverse primers bind. The primers act to delimit the region of the original template polynucleotide which is exponentially amplified during amplification. In some embodiments, additional primers may bind to the region 5' of the forward primer and/or reverse primers. Where such additional primers are used, the forward primer binding site and/or the reverse primer binding site may encompass the binding regions of these additional primers as well as the binding regions of the primers themselves. For example, in some embodiments, the method may use one or more additional primers which bind to a region that lies 5' of the forward and/or reverse primer binding region. Such a method was disclosed, for example, in WO0028082 which discloses the use of "displacement primers" or "outer primers."

A "barcode" nucleic acid identification sequence can be incorporated into a nucleic acid primer or linked to a primer to enable independent sequencing and identification to be associated with one another via a barcode which relates information and identification that originated from molecules that existed within the same sample. There are numerous techniques that can be used to attach barcodes to the nucleic acids within a discrete entity. For example, the target nucleic acids may or may not be first amplified and fragmented into shorter pieces. The molecules can be combined with discrete entities, e.g., droplets, containing the barcodes. The barcodes can then be attached to the molecules using, for example, splicing by overlap extension. In this approach, the initial target molecules can have "adaptor" or "constant" sequences added, which are molecules of a known sequence to which primers can be synthesized. When combined with the barcodes, primers can be used that are complementary to the adaptor sequences and the barcode sequences, such that the product amplicons of both target nucleic acids and barcodes can anneal to one another and, via an extension reaction such as DNA polymerization, be extended onto one another, generating a double- stranded product including the target nucleic acids attached to the barcode sequence. Alternatively, the primers that amplify that target can themselves be barcoded so that, upon annealing and extending onto the target, the amplicon produced has the barcode sequence incorporated into it. This can be applied with a number of amplification strategies, including specific amplification with PCR or non-specific amplification with, for example, MDA. An alternative enzymatic reaction that can be used to attach barcodes to nucleic acids is ligation, including blunt or sticky end ligation. In this approach, the DNA barcodes are incubated with the nucleic acid targets and ligase enzyme, resulting in the ligation of the barcode to the targets. The ends of the nucleic acids can be modified as needed for ligation by a number of techniques, including by using adaptors introduced with ligase or fragments to enable greater control over the number of barcodes added to the end of the molecule.

The terms "identity" and "identical" and their variants, as used herein, when used in reference to two or more sequences, refer to the degree to which the two or more sequences (e.g., nucleotide or polypeptide sequences) are the same. In the context of two or more sequences, the percent identity or homology of the sequences or subsequences thereof indicates the percentage of all monomeric units (e.g., nucleotides or amino acids) that are the same at a given position or region of the sequence (i.e., about 70% identity, preferably 75%, 80%, 85%, 90%, 95%, 97%, 98% or 99% identity). The percent identity can be over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

The terms "nucleic acid," "polynucleotides," and "oligonucleotides" refer to biopolymers of nucleotides and, unless the context indicates otherwise, includes modified and unmodified nucleotides, and both DNA and RNA, and modified nucleic acid backbones. For example, in certain embodiments, the nucleic acid is a peptide nucleic acid (PNA) or a locked nucleic acid (LNA). Typically, the methods as described herein are performed using DNA as the nucleic acid template for amplification. However, nucleic acid whose nucleotide is replaced by an artificial derivative or modified nucleic acid from natural DNA or RNA is also included in the nucleic acid of the present invention insofar as it functions as a template for synthesis of complementary chain. The nucleic acid of the present invention is generally contained in a biological sample. The biological sample includes animal, plant or microbial tissues, cells, cultures and excretions, or extracts therefrom. In certain aspects, the biological sample includes intracellular parasitic genomic DNA or RNA such as virus or mycoplasma. The nucleic acid may be derived from nucleic acid contained in said biological sample. For example, genomic DNA, or cDNA synthesized from mRNA, or nucleic acid amplified on the basis of nucleic acid derived from the biological sample, are preferably used in the described methods. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes deoxythymidine, and "U' denotes uridine. Oligonucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

A template nucleic acid is a nucleic acid serving as a template for synthesizing a complementary chain in a nucleic acid amplification technique. A complementary chain having a nucleotide sequence complementary to the template has a meaning as a chain corresponding to the template, but the relationship between the two is merely relative. That is, according to the methods described herein a chain synthesized as the complementary chain can function again as a template. That is, the complementary chain can become a template. In certain embodiments, the template is derived from a biological sample, e.g., plant, animal, virus, micro-organism, bacteria, fungus, etc. In certain embodiments, the animal is a mammal, e.g., a human patient. A template nucleic acid typically comprises one or more target nucleic acid. A target nucleic acid in exemplary embodiments may comprise any single or double-stranded nucleic acid sequence that can be amplified or synthesized according to the disclosure, including any nucleic acid sequence suspected or expected to be present in a sample.

Primers and oligonucleotides used in embodiments herein comprise nucleotides. In some embodiments, a nucleotide may comprise any compound, including without limitation any naturally occurring nucleotide or analog thereof, which can bind selectively to, or can be polymerized by, a polymerase. Typically, but not necessarily, selective binding of the nucleotide to the polymerase is followed by polymerization of the nucleotide into a nucleic acid strand by the polymerase; occasionally however the nucleotide may dissociate from the polymerase without becoming incorporated into the nucleic acid strand, an event referred to herein as a "non-productive" event. Such nucleotides include not only naturally occurring nucleotides but also any analogs, regardless of their structure, that can bind selectively to, or can be polymerized by, a polymerase. While naturally occurring nucleotides typically comprise base, sugar and phosphate moieties, the nucleotides of the present disclosure can include compounds lacking any one, some, or all of such moieties. For example, the nucleotide can optionally include a chain of phosphorus atoms comprising three, four, five, six, seven, eight, nine, ten or more phosphorus atoms. In some embodiments, the phosphorus chain can be attached to any carbon of a sugar ring, such as the 5' carbon. The phosphorus chain can be linked to the sugar with an intervening O or S. In one embodiment, one or more phosphorus atoms in the chain can be part of a phosphate group having P and O. In another embodiment, the phosphorus atoms in the chain can be linked together with intervening O, NH, S, methylene, substituted methylene, ethylene, substituted ethylene, CNH₂, C(O), C(CH₂), CH₂CH₂, or C(OH)CH₂R (where R can be a 4-pyridine or 1-imidazole). In one embodiment, the phosphorus atoms in the chain can have side groups having O, BH3, or S. In the phosphorus chain, a phosphorus atom with a side group other than O can be a substituted phosphate group. In the phosphorus chain, phosphorus atoms with an intervening atom other than O can be a substituted phosphate group. Some examples of nucleotide analogs are described in Xu, U.S. Pat. No. 7,405,281.

In some embodiments, the nucleotide comprises a label and referred to herein as a "labeled nucleotide"; the label of the labeled nucleotide is referred to herein as a "nucleotide label." In some embodiments, the label can be in the form of a fluorescent moiety (e.g. dye), luminescent moiety, or the like attached to the terminal phosphate group, i.e., the phosphate group most distal from the sugar. Some examples of nucleotides that can be used in the disclosed methods and compositions include, but are not limited to, ribonucleotides, deoxyribonucleotides, modified ribonucleotides, modified deoxyribonucleotides, ribonucleotide polyphosphates, deoxyribonucleotide polyphosphates, modified ribonucleotide polyphosphates, modified deoxyribonucleotide polyphosphates, peptide nucleotides, modified peptide nucleotides, metallonucleosides, phosphonate nucleosides, and modified phosphate-sugar backbone nucleotides, analogs, derivatives, or variants of the foregoing compounds, and the like. In some embodiments, the nucleotide can comprise non-oxygen moieties such as, for example, thio- or borano-moieties, in place of the oxygen moiety bridging the alpha phosphate and the sugar of the nucleotide, or the alpha and beta phosphates of the nucleotide, or the beta and gamma phosphates of the nucleotide, or between any other two phosphates of the nucleotide, or any combination thereof. "Nucleotide 5'- triphosphate" refers to a nucleotide with a triphosphate ester group at the 5' position, and are sometimes denoted as "NTP" or "dNTP" and "ddNTP" to particularly point out the structural features of the ribose sugar. The triphosphate ester group can include sulfur substitutions for the various oxygens, e.g. α-thio- nucleotide 5'-triphosphates. For a review of nucleic acid chemistry, see: Shabarova, Z. and Bogdanov, A. Advanced Organic Chemistry of Nucleic Acids, VCH, New York, 1994.

The phrase "tagmentation" refers to the process in which genomic DNA is cleaved, tagged with adapter sequences, and extended to fill in gaps arising from the cleavage and tagging. In various embodiments, cell lysis, genomic DNA release, and tagmentation within a single droplet. In various embodiments, tagmentation and nucleic acid amplification occur within a single droplet. The phrase "tagmented DNA" or "tagmented genomic DNA" refers to genomic DNA fragments following tagmentation. For example, tagmented DNA refers to cleaved DNA fragments including adaptor sequences and have further undergone nucleic acid extension to fill in gaps arising from the cleavage and tagging.

### Overview

Described herein are embodiments of a single-cell analysis workflow involving whole genome amplification for developing DNA libraries that cover the whole genome. Generally, the single-cell analysis workflow involves encapsulating and lysing cells in individual droplets and releasing genomic DNA from chromatin within the droplet. The released genomic DNA, which represents the whole genome of the cell, can be accessed by transposases, which cleave the genomic DNA and insert adaptor sequences into nucleic acid fragments that span the whole genome. Thus, the nucleic acid fragments can undergo nucleic acid amplification and sequencing for generating whole genome DNA libraries. Altogether, the implementation of the disclosed single-cell analysis workflow achieves improved library metrics (e.g. improved coverage across the whole genome, improved percentage of reads with correct structure, improved percentage of mapped reads, increased library complexity, increased library size, increased number of examined reads, and/or reduced number of duplicated reads).

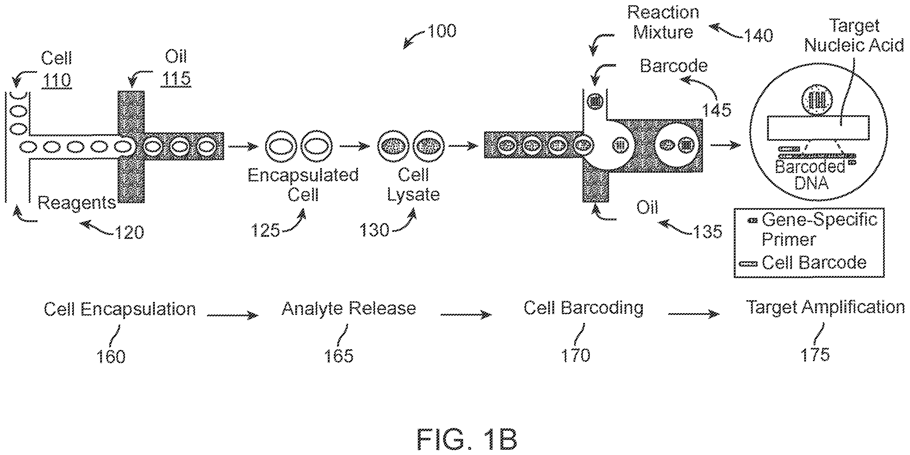

Figure (FIG.) 1A shows an overall system environment for analyzing cell(s) through a single cell workflow analysis, in accordance with an embodiment. Generally, the single cell workflow device 100 is configured to process the cell(s) 110 and generate sequence reads derived from individual cell(s) 110. Further details as to the processes of the single cell workflow device 100 are described below in reference to FIG. 1B. The computing device 180 can analyze the sequence reads e.g., for purposes of building libraries (e.g., whole genome DNA libraries) and/or characterizing individual cells. In various embodiments, the single cell workflow device 100 includes at least a microfluidic device that is configured to encapsulate cells with reagents to generate cell lysates comprising gDNA, perform tagmentation on the gDNA, and perform nucleic acid amplification reactions. For example, the microfluidic device can include one or more fluidic channels that are fluidically connected. Therefore, the combining of an aqueous fluid through a first channel and a carrier fluid through a second channel results in the generation of emulsion droplets. In various embodiments, the fluidic channels of the microfluidic device may have at least one cross-sectional dimension on the order of a millimeter or smaller (e.g., less than or equal to about 1 millimeter). Additional details of microchannel design and dimensions is described in International Patent Application No. PCT/US2016/016444 and US Patent Application Publication No. US 2015/0232942 A1. An example of a microfluidic device is the Tapestri^{™} Platform.

In various embodiments, the single cell workflow device 100 may also include one or more of: (a) a temperature control module for controlling the temperature of one or more portions of the subject devices and/or droplets therein and which is operably connected to the microfluidic device(s), (b) a detection means, i.e., a detector, e.g., an optical imager, operably connected to the microfluidic device(s), (c) an incubator, e.g., a cell incubator, operably connected to the microfluidic device(s), and (d) a sequencer operably connected to the microfluidic device(s). The one or more temperature and/or pressure control modules provide control over the temperature and/or pressure of a carrier fluid in one or more flow channels of a device. As an example, a temperature control module may be one or more thermal cycler that regulates the temperature for performing nucleic acid amplification. The one or more detection means i.e., a detector, e.g., an optical imager, are configured for detecting the presence of one or more droplets, or one or more characteristics thereof, including their composition. In some embodiments, detection means are configured to recognize one or more components of one or more droplets, in one or more flow channel. The sequencer is a hardware device configured to perform sequencing, such as next generation sequencing. Examples of sequencers include Illumina sequencers (e.g., MiniSeq^{™}, MiSeq^{™}, NextSeq^{™} 550 Series, or NextSeq^{™} 2000), Roche sequencing system 454, and Thermo Fisher Scientific sequencers (e.g., Ion GeneStudio S5 system, Ion Torrent Genexus System).

### Methods for Performing Single-Cell Analysis

### Encapuslation, Analyte Release, Barcoding, and Amplification

Embodiments described herein involve encapsulating one or more cells to perform single-cell analysis on the one or more cells. As described herein, the single-cell analysis can involve whole genome amplification for sequencing and analysis of the whole genome of a subject or patient.

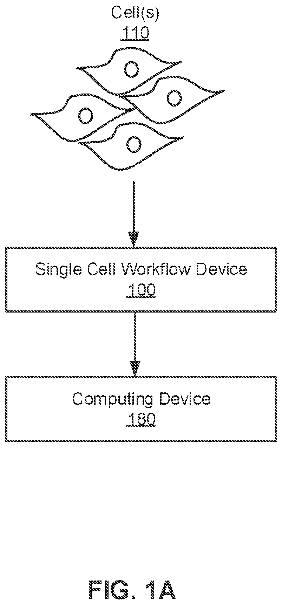

Reference is now made to FIG. 1B, which depicts an embodiment of processing single cells to generate amplified nucleic acid molecules for sequencing. Here, the processing of single cells can be performed by a single cell workflow device (e.g., the single cell workflow device 100 disclosed in FIG. 1A). Specifically, FIG. 1B depicts a workflow process including the steps of cell encapsulation 160, analyte release 165, cell barcoding 170, and target amplification 175 of target nucleic acid molecules.

As referred herein, the workflow process shown in FIG. 1B is a two-step workflow process in which analyte release 165 from the cell occurs separate from the steps of cell barcoding 170 and target amplification 175. Specifically, analyte release 165 from a cell occurs within a first droplet followed by cell barcoding 170 and target amplification 175 in a second emulsion. As described in further detail below, tagmentation of genomic DNA can occur in either the first droplet, or in the second droplet. In various embodiments, alternative workflow processes (e.g., workflow processes other than the two-step workflow process shown in FIG. 1A) can be employed. For example, the cell 110, reagents 120, reaction mixture 140, and barcode 145 can be encapsulated in a single emulsion. Thus, analyte release 165 can occur within the droplet, followed by cell barcoding 170 and target amplification 175 within the same droplet. Here, tagmentation of genomic DNA can also occur in the same droplet. Additionally, although FIG. 1B depicts cell barcoding 170 and target amplification 175 as two separate steps, in various embodiments, the target nucleic acid is labeled with a barcode 145 through the nucleic acid amplification step.

Generally, the cell encapsulation step 160 involves encapsulating a single cell 110 with reagents 120 into a droplet. In various embodiments, single cells 110 can be isolated from a test sample obtained from a subject or a patient. In various embodiments, single cells 110 are healthy cells taken from a healthy subject. Thus, the single-cell analysis enables whole genome analysis of the healthy subject.

In various embodiments, single cells 110 include cells taken from a subject previously diagnosed with disease. Thus, the single-cell analysis enables whole genome analysis of the diseased subject. In various embodiments, the subject is previously diagnosed with cancer. Here, single-cell analysis can be performed on one or more tumor cells obtained from the subject diagnosed with cancer. Thus, single-cell analysis of the tumor cells enables whole genome analysis of the subject's cancer.

In various embodiments, the droplet is formed by partitioning aqueous fluid containing the cell 110 and reagents 120 into a carrier fluid (e.g., oil 115), thereby resulting in a aqueous fluid-in-oil emulsion. In various embodiments, encapsulating a cell 110 with reagents 120 is accomplished by combining an aqueous phase including the cell 110 and reagents 120 with an immiscible oil phase. In one embodiment, an aqueous phase including the cell 110 and reagents 120 are flowed together with a flowing immiscible oil phase such that water in oil emulsions are formed, where at least one emulsion includes a single cell and the reagents. In various embodiments the immiscible oil phase includes a fluorous oil, a fluorous non-ionic surfactant, or both. In various embodiments, emulsions can have an internal volume of about 0.001 to 1000 picoliters or more and can range from 0.1 to 1000 µm in diameter.

In various embodiments, the aqueous phase including the cell and reagents need not be simultaneously flowing with the immiscible oil phase. For example, the aqueous phase can be flowed to contact a stationary reservoir of the immiscible oil phase, thereby enabling the budding of water in oil emulsions within the stationary oil reservoir.

In various embodiments, combining the aqueous phase and the immiscible oil phase can be performed in a microfluidic device. For example, the aqueous phase can flow through a microchannel of the microfluidic device to contact the immiscible oil phase, which is simultaneously flowing through a separate microchannel or is held in a stationary reservoir of the microfluidic device. The encapsulated cell and reagents within an emulsion can then be flowed through the microfluidic device to undergo cell lysis.

Further example embodiments of adding reagents and cells to emulsions can include merging emulsions that separately contain the cells and reagents or picoinjecting reagents into an emulsion. Further description of example embodiments is described in US Application Publication No. US 2015/0232942 A1.

The droplet includes encapsulated cell 125 and the reagents 120. The encapsulated cell undergoes an analyte release at step 165. Generally, the reagents cause the cell to lyse, thereby generating a cell lysate 130 within the droplet. Thus, the cell lysate 130 includes the contents of the cell, which can include one or more different types of analytes (e.g., RNA transcripts, DNA, protein, lipids, or carbohydrates).

In various embodiments, the cell is lysed due to the reagents which include one or more lysing agents that cause the cell to lyse. Examples of lysing agents include detergents such as Triton X-100, NP-40 (e.g., Tergitol-type NP-40 or nonyl phenoxypolyethoxylethanol), as well as cytotoxins. Examples of NP-40 include Thermo Scientific NP-40 Surfact-Amps Detergent solution and Sigma Aldrich NP-40 (TERGITOL Type NP-40). In some embodiments, cell lysis may also, or instead, rely on techniques that do not involve a lysing agent in the reagent. For example, lysis may be achieved by mechanical techniques that may employ various geometric features to effect piercing, shearing, abrading, etc. of cells. Other types of mechanical breakage such as acoustic techniques may also be used. Further, thermal energy can also be used to lyse cells. Any convenient means of effecting cell lysis may be employed in the methods described herein.

In various embodiments, the reagents can cause release of the genomic DNA from chromatin packaging. For example, the reagents can include a protease that digests chromatin packaging, thereby freeing the genomic DNA for subsequent processing. In various embodiments, the protease is proteinase K.

In various embodiments, the reagents 120 include agents for performing tagmentation on the released genomic DNA. In such embodiments, tagmentation is performed in the first droplet. For example, the reagents 120 can include transposases that cleave the genomic DNA into fragments that span the whole genome. In various embodiments, the transposases are linked to adaptor sequences. Thus, the transposases can insert the adaptor sequences into the fragments.

In various embodiments, the reagents 120 include enzymes for interacting with nucleic acids of the cell lysate. In various embodiments, the reagents 120 include reverse transcriptase. For example, reverse transcriptase can reverse transcribe RNA transcripts that are present in the cell lysate 130 and generate cDNA molecules. Here, the generation of cDNA enables the subsequent analysis of amplicons derived from the RNA transcripts and further allows analysis and characterization of single cell transcriptomes. As another example, in embodiments where tagmentation is performed in the first droplet, reverse transcriptase can extend DNA fragments with adaptor sequences that have been inserted by transposases. Thus, reverse transcriptase fills in any gaps in the DNA fragments that may have been created by inserting the adaptor sequences. In various embodiments, the reagents 120 include DNA polymerase. For example, in embodiments where tagmentation is performed in the first droplet, DNA polymerase is included in the reagents 120 for extending DNA fragments with adaptor sequences. Thus, DNA polymerase fills in any gaps in the DNA fragments that may have been created by inserting the adaptor sequences.

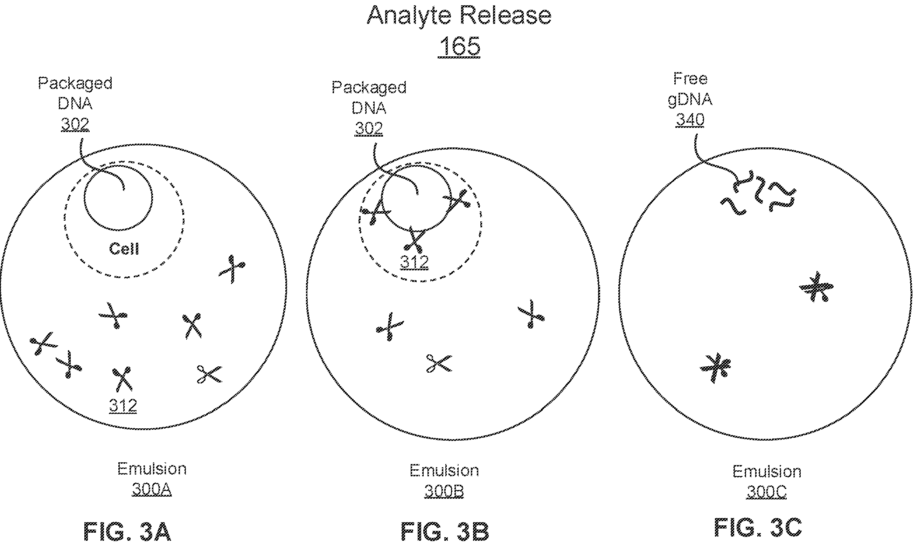

FIGs. 3A-3C depict the processing and releasing of analytes of a single cell in a droplet, in accordance with an embodiment. In FIG. 3A, the cell is lysed, as indicated by the dotted line of the cell membrane. In some embodiments, the reagents include a detergent, such as NP40 or Triton-X100, which causes the cell to lyse. The lysed cell includes packaged DNA 302, which refers to the organization of genomic DNA with histones that are packaged as chromatin. Furthermore, the reagents included in the emulsion 300A further includes an enzyme 312 that digests the packaged DNA 302. In various embodiments, the enzyme 312 is proteinase K.

FIG. 3B depicts the emulsion 300B in a second state as the enzymes 312 digest the packaged DNA 302, thereby causing release of genomic DNA. FIG. 3C depicts the emulsion 300C in a third state that includes the free gDNA 340.

In various embodiments, the emulsion 300C can be exposed to conditions to inactivate the enzymes 312. In various embodiments, the emulsion 300C is exposed to an elevated temperature of at least 50°C to inactivate the enzymes 312. In various embodiments, the emulsion 300C is exposed to an elevated temperature of at least 60°C to inactivate the enzymes 312. In various embodiments, the emulsion 300C is exposed to an elevated temperature of at least 70°C to inactivate the enzymes 312. In various embodiments, the emulsion 300C is exposed to an elevated temperature of at least 80°C to inactivate the enzymes 312. In various embodiments, the emulsion 300C is exposed to an elevated temperature of at least 90°C to inactivate the enzymes 312.

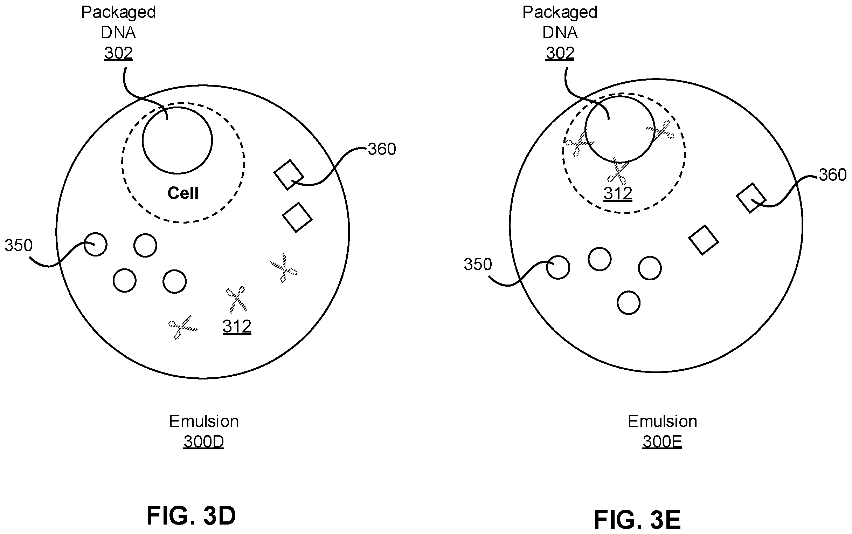

In contrast to FIGs. 3A-3C, FIGs. 3D-3G depict the processing and releasing of analytes of a single cell in a droplet, in accordance with an embodiment in which tagmentation is performed in a first droplet. In FIG. 3D, the emulsion 300D includes the cell, packaged DNA 302 within the cell, enzyme 312 for releasing gDNA, transposases 350, and an enzyme 360 for nucleic acid synthesis (e.g., enzyme 360 is reverse transcriptase or DNA polymerase). As shown in FIG. 3D, the cell is lysed, as indicated by the dotted line of the cell membrane. In some embodiments, the reagents include a detergent, such as NP40 or Triton-X100, which causes the cell to lyse.

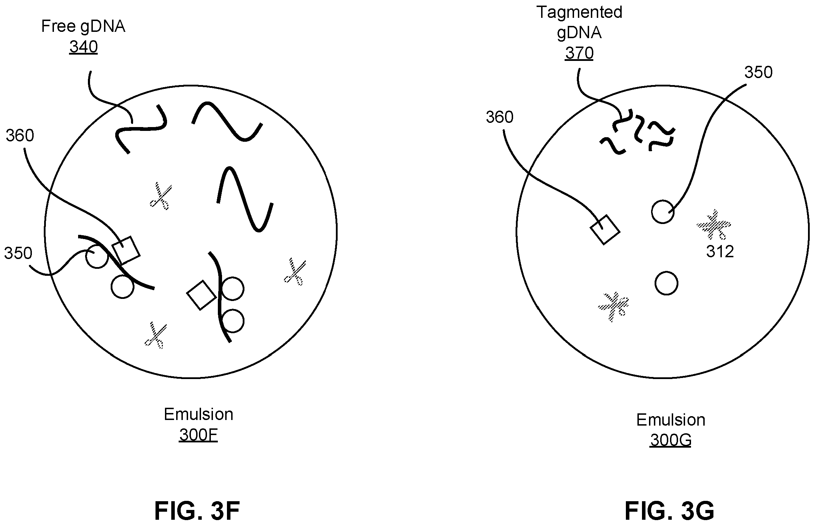

FIG. 3E depicts the emulsion 300E in a second state as the enzymes 312 digest the packaged DNA 302. FIG. 3F depicts the emulsion 300F in a third state that includes the free gDNA 340. Here, tagmentation can be performed on the free gDNA 340. For example, transposases 350 can cleave free gDNA 340 and insert adaptor sequences. The enzyme 360 performs an extension to fill in gaps in the cleaved genomic DNA resulting from the insertion of adaptor sequences. Although FIG. 3F depicts a pair of transposases 350 forming a complex on a gDNA 340, in various embodiments, a single free gDNA 340 can be recognized by additional transposases, thereby enabling cleavage of the gDNA 340 into smaller fragments. For example, a single free gDNA 340 can be recognized by tens, hundreds, thousands, tens of thousands, hundreds of thousands, or even millions of transposases.

As shown in FIG. 3G, emulsion 300G includes the tagmented gDNA 370, which represents cleaved genomic DNA including the inserted adaptor sequences. In various embodiments, the emulsion 300G can be exposed to conditions to inactivate the enzymes 312. In various embodiments, the emulsion 300G is exposed to an elevated temperature of at least 50°C to inactivate the enzymes 312. In various embodiments, the emulsion 300G is exposed to an elevated temperature of at least 60°C to inactivate the enzymes 312. In various embodiments, the emulsion 300G is exposed to an elevated temperature of at least 70°C to inactivate the enzymes 312. In various embodiments, the emulsion 300G is exposed to an elevated temperature of at least 80°C to inactivate the enzymes 312. In various embodiments, the emulsion 300G is exposed to an elevated temperature of at least 90°C to inactivate the enzymes 312. In various embodiments, the emulsion 300G is exposed to an elevated temperature of at least 95°C to inactivate the enzymes 312.

Returning to the step of cell barcoding 170 in FIG. 1B, the cell barcoding step 170 involves encapsulating the cell lysate 130 into a second droplet along with a barcode 145 and/or reaction mixture 140. In various embodiments, a cell lysate 130 is encapsulated with a reaction mixture 140 and a barcode 145 by combining an aqueous phase including the reaction mixture 140 and the barcode 145 with the cell lysate 130 and an immiscible oil phase 135. As shown in FIG. 1B, the reaction mixture 140 and barcode 145 can be introduced through a separate stream of aqueous fluid, thereby partitioning the reaction mixture 140 and barcode 145 into the second droplet along with the cell lysate 130. In various embodiments, an aqueous phase including the reaction mixture 140 and the barcode 145 are flowed together with a flowing cell lysate 130 and a flowing immiscible oil phase 135 such that water in oil emulsions are formed, where at least one emulsion includes a cell lysate 130, the reaction mixture 140, and the barcode 145. In various embodiments the immiscible oil phase includes a fluorous oil, a fluorous non-ionic surfactant, or both. In various embodiments, emulsions can have an internal volume of about 0.001 to 1000 picoliters or more and can range from 0.1 to 1000 µm in diameter.

In various embodiments, combining the aqueous phase and the immiscible oil phase can be performed in a microfluidic device. For example, the aqueous phase can flow through a microchannel of the microfluidic device to contact the immiscible oil phase, which is simultaneously flowing through a separate microchannel or is held in a stationary reservoir of the microfluidic device. The encapsulated cell lysate, reaction mixture, and barcode within an emulsion can then be flowed through the microfluidic device to perform amplification of target nucleic acids.

Further example embodiments of adding reaction mixture and barcodes to emulsions can include merging emulsions that separately contain the cell lysate and reaction mixture and barcodes or picoinjecting the reaction mixture and/or barcode into an emulsion. Further description of example embodiments of merging emulsions or picoinjecting substances into an emulsion is found in US Application Publication No. US 2015/0232942 A1.

Generally, the reaction mixture includes reactants sufficient for performing a reaction, such as nucleic acid amplification, on analytes of the cell lysate. In various embodiments, the reaction mixture 140 includes components, such as primers, for performing the nucleic acid reaction on the analytes. Such primers are capable of acting as a point of initiation of synthesis along a complementary strand when placed under conditions in which synthesis of a primer extension product which is complementary to a nucleic acid strand is catalyzed.

In various embodiments, the reaction mixture 140 enables the tagmentation of genomic DNA in a cell lysate 130. Here, tagmentation is performed in the second droplet and is not performed in the first droplet (e.g., droplet involving cell lysis and genomic DNA release). For example, the reaction mixture 140 can include transposases that cleave the genomic DNA into fragments that span the whole genome. In various embodiments, the transposases are linked to adaptor sequences. Thus, the transposases can insert the adaptor sequences into the fragments. In various embodiments, the reaction mixture 140 include DNA polymerase. For example, in embodiments where tagmentation is performed in the second droplet, DNA polymerase is included in the reaction mixture 140 for extending DNA fragments with adaptor sequences. Thus, DNA polymerase fills in any gaps in the DNA fragments that may have been created by inserting the adaptor sequences.

The target amplification step 175 involves amplifying target nucleic acids. For example, target nucleic acids of the cell lysate undergo amplification using the reaction mixture 140 in the second droplet, thereby generating amplicons derived from the target nucleic acids. In various embodiments, the target nucleic acids include tagmented genomic DNA with the adaptor sequences. The tagmented genomic DNA spans the whole genome and therefore, nucleic acid amplification results in the generation of amplicons that span the whole genome.

The emulsion may be incubated under conditions that facilitates the nucleic acid amplification reaction. In various embodiments, the emulsion may be incubated on the same microfluidic device as was used to add the reaction mixture and/or barcode, or may be incubated on a separate device. In certain embodiments, incubating the emulsion under conditions that facilitates nucleic acid amplification is performed on the same microfluidic device used to encapsulate the cells and lyse the cells. Incubating the emulsions may take a variety of forms. In certain aspects, the emulsions containing the reaction mix, barcode, and cell lysate may be flowed through a channel that incubates the emulsions under conditions effective for nucleic acid amplification. Flowing the microdroplets through a channel may involve a channel that snakes over various temperature zones maintained at temperatures effective for PCR. Such channels may, for example, cycle over two or more temperature zones, wherein at least one zone is maintained at about 65° C. and at least one zone is maintained at about 95° C. As the drops move through such zones, their temperature cycles, as needed for nucleic acid amplification. The number of zones, and the respective temperature of each zone, may be readily determined by those of skill in the art to achieve the desired nucleic acid amplification. Additionally, the extent of nucleic amplification can be controlled by modulating the concentration of the reactants in the reaction mixture. In some instances, this is useful for fine tuning of the reactions in which the amplified products are used.

In various embodiments, the nucleic amplification reaction involves incorporating a barcode 145 into an amplicon, such as a target nucleic acid to be analyzed (e.g., a tagmented genomic DNA), which enables subsequent identification of the origin of a sequence read that is derived from the target nucleic acid. In various embodiments, multiple barcodes 145 can label multiple amplicons (e.g., target nucleic acids of the cell lysate), thereby enabling the subsequent identification of the origin of large quantities of sequence reads.

### Pooling, Sequencing and Read Alignment

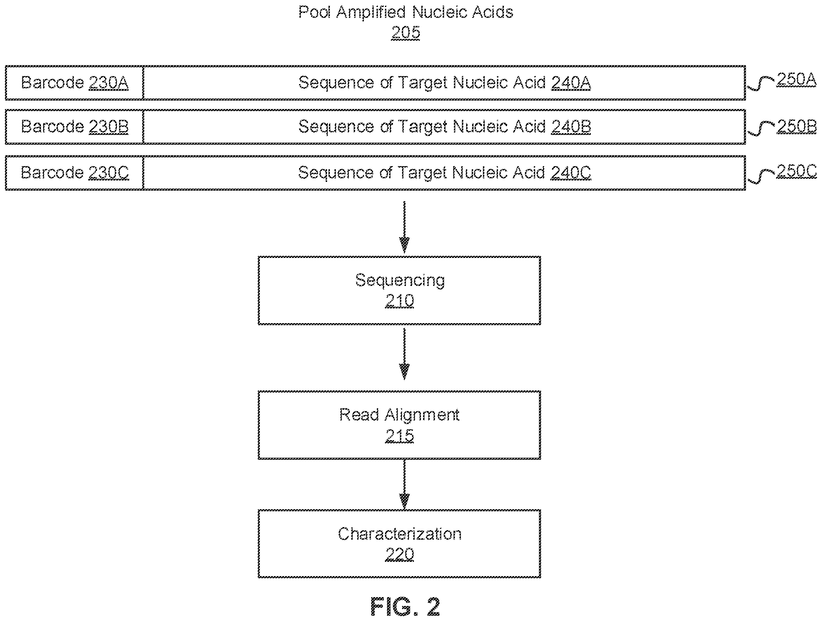

FIG. 2 is a flow process for analyzing nucleic acid sequences derived from analytes of the single cell, in accordance with an embodiment. Specifically, FIG. 2 depicts the steps of pooling amplified nucleic acids at step 205, sequencing the amplified nucleic acids at step 210, read alignment at step 215, and characterization at step 220. Generally, the flow process shown in FIG. 2 is a continuation of the workflow process shown in FIG. 1B.

For example, after target amplification at step 175 of FIG. 1B, the amplified nucleic acids 250A, 250B, and 250C are pooled at step 205 shown in FIG. 2. For example, individual droplets containing amplified nucleic acids are pooled and collected, and the immiscible oil of the emulsions is removed. In various embodiments, the droplets are collected in a well, such as a well of a microfluidic device. In various embodiments, the droplets are collected in a reservoir or a tube, such as an Eppendorf tube. In one embodiment, the emulsions are broken by providing an external stimuli to pool the amplified nucleic acids. In one embodiment, the emulsions naturally aggregate over time given the density differences between the aqueous phase and immiscible oil phase. Thus, amplified nucleic acids from multiple cells can be pooled together.

In various embodiments, the pooled nucleic acids can undergo further preparation for sequencing. For example, sequencing adapters can be added to the pooled nucleic acids. Example sequencing adapters are P5 and P7 sequencing adapters. The sequencing adapters enable the subsequent sequencing of the nucleic acids. In various embodiments, incorporation of the sequencing adapters includes performing a library amplification step.

FIG. 2 depicts three amplified nucleic acids 250A, 250B, and 250C. In various embodiments, pooled nucleic acids can include hundreds, thousands, or millions of nucleic acids derived from analytes of multiple cells. In various embodiments, the amplified nucleic acids in the pool are derived from tagmented genomic DNA. In such embodiments, the amplified nucleic acids in the pool can span the whole genome.

In various embodiments, each amplified nucleic acid 250 includes at least a sequence of a target nucleic acid 240 and a barcode 230. In various embodiments, an amplified nucleic acid 250 can include additional sequences, such as any of a universal primer sequence, a random primer sequence, a gene specific primer forward sequence, a gene specific primer reverse sequence, a constant region, or sequencing adapters.

In various embodiments, the amplified nucleic acids 250A, 250B, and 250C are derived from the same single cell and therefore, the barcodes 230A, 230B, and 230C are the same. Therefore, sequencing of the barcodes 230 enables the determination that the amplified nucleic acids 250 are derived from the same cell. In various embodiments, the amplified nucleic acids 250A, 250B, and 250C are pooled and derived from different cells. Therefore, the barcodes 230A, 230B, and 230C are different from one another and sequencing of the barcodes 230 enables the determination that the amplified nucleic acids 250 are derived from different cells.

At step 210, the pooled amplified nucleic acids 250 undergo sequencing to generate sequence reads. For each of one or more amplicons, the sequence read includes at least the sequence of the barcode and the target nucleic acid. Sequence reads originating from individual cells are clustered according to the barcode sequences included in the amplicons. Amplified nucleic acids are sequenced to obtain sequence reads for generating a sequencing library. Sequence reads can be achieved with commercially available next generation sequencing (NGS) platforms, including platforms that perform any of sequencing by synthesis, sequencing by ligation, pyrosequencing, using reversible terminator chemistry, using phospholinked fluorescent nucleotides, or real-time sequencing. As an example, amplified nucleic acids may be sequenced on an Illumina MiSeq platform.

When pyrosequencing, libraries of NGS fragments are cloned in-situ amplified by capture of one matrix molecule using granules coated with oligonucleotides complementary to adapters. Each granule containing a matrix of the same type is placed in a microbubble of the "water in oil" type and the matrix is cloned amplified using a method called emulsion PCR. After amplification, the emulsion is destroyed and the granules are stacked in separate wells of a titration picoplate acting as a flow cell during sequencing reactions. The ordered multiple administration of each of the four dNTP reagents into the flow cell occurs in the presence of sequencing enzymes and a luminescent reporter, such as luciferase. In the case where a suitable dNTP is added to the 3 ' end of the sequencing primer, the resulting ATP produces a flash of luminescence within the well, which is recorded using a CCD camera. It is possible to achieve a read length of more than or equal to 400 bases, and it is possible to obtain 10⁶ readings of the sequence, resulting in up to 500 million base pairs (megabytes) of the sequence. Additional details for pyrosequencing is described in Voelkerding et al., Clinical Chem., 55: 641-658, 2009; MacLean et al., Nature Rev. Microbiol., 7: 287-296; US patent No. 6,210,891; US patent No. 6,258,568.

On the Solexa / Illumina platform, sequencing data is produced in the form of short readings. In this method, fragments of a library of NGS fragments are captured on the surface of a flow cell that is coated with oligonucleotide anchor molecules. An anchor molecule is used as a PCR primer, but due to the length of the matrix and its proximity to other nearby anchor oligonucleotides, elongation by PCR leads to the formation of a "vault" of the molecule with its hybridization with the neighboring anchor oligonucleotide and the formation of a bridging structure on the surface of the flow cell. These DNA loops are denatured and cleaved. Straight chains are then sequenced using reversibly stained terminators. The nucleotides included in the sequence are determined by detecting fluorescence after inclusion, where each fluorescent and blocking agent is removed prior to the next dNTP addition cycle. Additional details for sequencing using the Illumina platform is found in Voelkerding et al., Clinical Chem., 55: 641-658, 2009; MacLean et al., Nature Rev. Microbiol., 7: 287-296; US patent No. 6,833,246; US patent No. 7,115,400; US patent No. 6,969,488.

Sequencing of nucleic acid molecules using SOLiD technology includes clonal amplification of the library of NGS fragments using emulsion PCR. After that, the granules containing the matrix are immobilized on the derivatized surface of the glass flow cell and annealed with a primer complementary to the adapter oligonucleotide. However, instead of using the indicated primer for 3 'extension, it is used to obtain a 5' phosphate group for ligation for test probes containing two probe-specific bases followed by 6 degenerate bases and one of four fluorescent labels. In the SOLiD system, test probes have 16 possible combinations of two bases at the 3 'end of each probe and one of four fluorescent dyes at the 5' end. The color of the fluorescent dye and, thus, the identity of each probe, corresponds to a certain color space coding scheme. After many cycles of alignment of the probe, ligation of the probe and detection of a fluorescent signal, denaturation followed by a second sequencing cycle using a primer that is shifted by one base compared to the original primer. In this way, the sequence of the matrix can be reconstructed by calculation; matrix bases are checked twice, which leads to increased accuracy. Additional details for sequencing using SOLiD technology is found in Voelkerding et al., Clinical Chem., 55: 641-658, 2009; MacLean et al., Nature Rev. Microbiol., 7: 287-296; US patent No. 5,912,148; US patent No. 6,130,073.

In particular embodiments, HeliScope from Helicos BioSciences is used. Sequencing is achieved by the addition of polymerase and serial additions of fluorescently-labeled dNTP reagents. Switching on leads to the appearance of a fluorescent signal corresponding to dNTP, and the specified signal is captured by the CCD camera before each dNTP addition cycle. The reading length of the sequence varies from 25-50 nucleotides with a total yield exceeding 1 billion nucleotide pairs per analytical work cycle. Additional details for performing sequencing using HeliScope is found in Voelkerding et al., Clinical Chem., 55: 641-658, 2009; MacLean et al., Nature Rev. Microbiol., 7: 287-296; US Patent No. 7,169,560; US patent No. 7,282,337; US patent No. 7,482,120; US patent No. 7,501,245; US patent No. 6,818,395; US patent No. 6,911,345; US patent No. 7,501,245.

In some embodiments, a Roche sequencing system 454 is used. Sequencing 454 involves two steps. In the first step, DNA is cut into fragments of approximately 300-800 base pairs, and these fragments have blunt ends. Oligonucleotide adapters are then ligated to the ends of the fragments. The adapter serve as primers for amplification and sequencing of fragments. Fragments can be attached to DNA-capture beads, for example, streptavidin-coated beads, using, for example, an adapter that contains a 5'-biotin tag. Fragments attached to the granules are amplified by PCR within the droplets of an oil-water emulsion. The result is multiple copies of cloned amplified DNA fragments on each bead. At the second stage, the granules are captured in wells (several picoliters in volume). Pyrosequencing is carried out on each DNA fragment in parallel. Adding one or more nucleotides leads to the generation of a light signal, which is recorded on the CCD camera of the sequencing instrument. The signal intensity is proportional to the number of nucleotides included. Pyrosequencing uses pyrophosphate (PPi), which is released upon the addition of a nucleotide. PPi is converted to ATP using ATP sulfurylase in the presence of adenosine 5 'phosphosulfate. Luciferase uses ATP to convert luciferin to oxyluciferin, and as a result of this reaction, light is generated that is detected and analyzed. Additional details for performing sequencing 454 is found in Margulies et al. (2005) Nature 437: 376-380.

Ion Torrent technology is a DNA sequencing method based on the detection of hydrogen ions that are released during DNA polymerization. The microwell contains a fragment of a library of NGS fragments to be sequenced. Under the microwell layer is the hypersensitive ion sensor ISFET. All layers are contained within a semiconductor CMOS chip, similar to the chip used in the electronics industry. When dNTP is incorporated into a growing complementary chain, a hydrogen ion is released that excites a hypersensitive ion sensor. If homopolymer repeats are present in the sequence of the template, multiple dNTP molecules will be included in one cycle. This results in a corresponding amount of hydrogen atoms being released and in proportion to a higher electrical signal. This technology is different from other sequencing technologies that do not use modified nucleotides or optical devices. Additional details for Ion Torrent Technology is found in Science 327 (5970): 1190 (2010); US Patent Application Publication Nos. 20090026082, 20090127589, 20100301398, 20100197507, 20100188073, and 20100137143.

In various embodiments, sequencing reads obtained from the NGS methods can be filtered by quality and grouped by barcode sequence using any algorithms known in the art, e.g., Python script barcodeCleanup.py . In some embodiments, a given sequencing read may be discarded if more than about 20% of its bases have a quality score (Q-score) less than Q20, indicating a base call accuracy of about 99%. In some embodiments, a given sequencing read may be discarded if more than about 5%, about 10%, about 15%, about 20%, about 25%, about 30% have a Q-score less than Q10, Q20, Q30, Q40, Q50, Q60, or more, indicating a base call accuracy of about 90%, about 99%, about 99.9%, about 99.99%, about 99.999%, about 99.9999%, or more, respectively.

In some embodiments, all sequencing reads associated with a barcode containing less than 50 reads may be discarded to ensure that all barcode groups, representing single cells, contain a sufficient number of high-quality reads. In some embodiments, all sequencing reads associated with a barcode containing less than 30, less than 40, less than 50, less than 60, less than 70, less than 80, less than 90, less than 100 or more may be discarded to ensure the quality of the barcode groups representing single cells.

At step 215, the sequence reads for each single cell are aligned (e.g., to a reference genome). Sequence reads with common barcode sequences (e.g., meaning that sequence reads originated from the same cell) may be aligned to a reference genome using known methods in the art to determine alignment position information. The alignment position information may indicate a beginning position and an end position of a region in the reference genome that corresponds to a beginning nucleotide base and end nucleotide base of a given sequence read. A region in the reference genome may be associated with a target gene or a segment of a gene. Example aligner algorithms include BWA, Bowtie, Spliced Transcripts Alignment to a Reference (STAR), Tophat, or HISAT2. Further details for aligning sequence reads to reference sequences is described in US Application Publication No. US 2020/0051663 A1. In various embodiments, an output file having SAM (sequence alignment map) format or BAM (binary alignment map) format may be generated and output for subsequent analysis.

Aligning the sequence reads to the reference genome enables the determination of where in the genome the sequence read is derived from. For example, multiple sequence reads generated from amplicons derived from a RNA transcript molecule, when aligned to a position of the genome, can reveal that a gene at the position of the genome was transcribed. As another example, multiple sequence reads generated amplicons derived from a genomic DNA molecule, when aligned to a position of the genome, can reveal the sequence of the gene at the position of the genome. The alignment of sequence reads at step 215 generates libraries, such as single cell DNA libraries or single cell RNA libraries. In various embodiments, the libraries are whole genome DNA libraries. Here, the aligned sequence reads at step 215 can span the whole genome.

At step 220, characterization of the libraries and/or the single cells can be performed. In various embodiments, sequencing and read alignment results in generation of a nucleic acid library (e.g., a RNA library and/or a DNA library). In particular embodiments, the nucleic acid library is a whole genome library. In various embodiments, characterization of a library (e.g., DNA library or RNA library) can involve determining library metrics including, but not limited to: read coverage across the whole genome, percentage of reads with correct structure, percentage of mapped reads, library complexity, library size, number of examined reads, and number of duplicated reads. In various embodiments, characterization of single cells can involve identifying one or more mutations (e.g., allelic variants, point mutations, single nucleotide variations/polymorphisms, translocations, DNA/RNA fusions, loss of heterozygosity) that are present in one or more of the single cells. Further description regarding characterization of single cells is described in PCT/US2020/026480 and PCT/US2020/026482.

In particular embodiments, the nucleic acid library is a whole genome library (e.g., whole genome sequencing library) that includes whole genome amplicons derived from genomic DNA across the chromosomes of a single human cell. In various embodiments, at least 20% of the plurality of sequence reads of the whole genome sequencing library are mapped to a reference genome. In various embodiments, at least 50% of the plurality of sequence reads of the whole genome sequencing library are mapped to a reference genome. In various embodiments, at least 80% of the plurality of sequence reads of the whole genome sequencing library are mapped to a reference genome. In various embodiments, at least 90% of the plurality of sequence reads of the whole genome sequencing library are mapped to a reference genome. In various embodiments, at least 95% of the plurality of sequence reads of the whole genome sequencing library are mapped to a reference genome. In various embodiments, at least 99% of the plurality of sequence reads of the whole genome sequencing library are mapped to a reference genome.

In various embodiments, at least 10% of sequence reads of the whole genome sequencing library have a correct structure. In various embodiments, at least 30% of sequence reads of the whole genome sequencing library have a correct structure. In various embodiments, at least 50% of sequence reads of the whole genome sequencing library have a correct structure. In various embodiments, at least 60% of sequence reads of the whole genome sequencing library have a correct structure. In various embodiments, at least 70% of sequence reads of the whole genome sequencing library have a correct structure. In various embodiments, at least 80% of sequence reads of the whole genome sequencing library have a correct structure. In various embodiments, at least 90% of sequence reads of the whole genome sequencing library have a correct structure. In various embodiments, at least 95% of sequence reads of the whole genome sequencing library have a correct structure. In various embodiments, at least 99% of sequence reads of the whole genome sequencing library have a correct structure.

In various embodiments, less than 70% of the sequences reads of the whole genome sequencing library are duplicated. In various embodiments, less than 60% of the sequences reads of the whole genome sequencing library are duplicated. In various embodiments, less than 50% of the sequences reads of the whole genome sequencing library are duplicated. In various embodiments, less than 40% of the sequences reads of the whole genome sequencing library are duplicated. In various embodiments, less than 30% of the sequences reads of the whole genome sequencing library are duplicated. In various embodiments, less than 20% of the sequences reads of the whole genome sequencing library are duplicated. In various embodiments, less than 10% of the sequences reads of the whole genome sequencing library are duplicated. In various embodiments, less than 5% of the sequences reads of the whole genome sequencing library are duplicated.

### Example Tagmentation

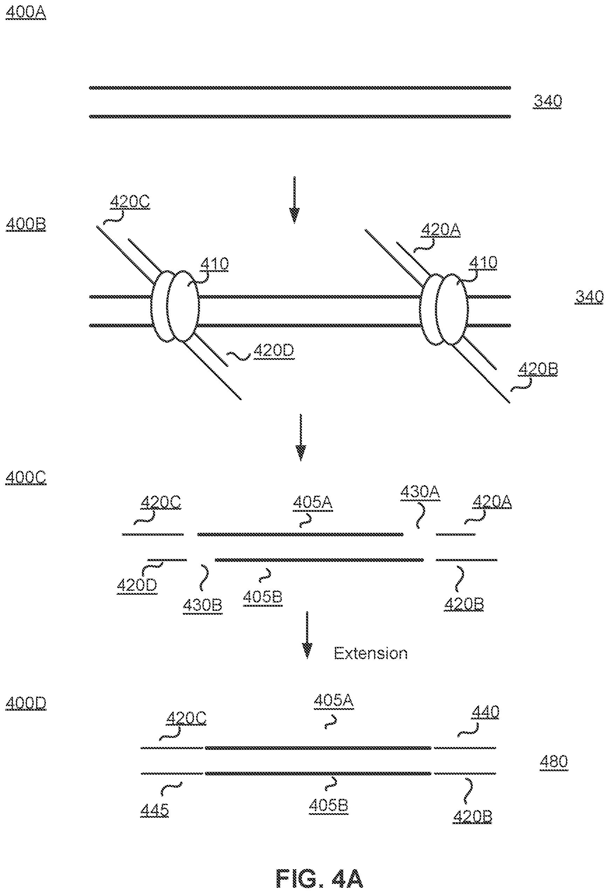

FIG. 4A depicts the tagmentation of genomic DNA, in accordance with an embodiment. In various embodiments, the steps shown in FIG. 4A are performed in a first droplet. For example, the tagmentation steps of FIG. 4A are performed during the cell encapsulation 160 and analyte release 165 steps shown in FIG. 1B. Thus, in such embodiments, tagmentation of the genomic DNA occurs in the presence of an enzyme for releasing genomic DNA from chromatin. For example, tagmentation of genomic DNA occurs in the presence of a protease, such as proteinase K. In various embodiments, the steps shown in FIG. 4A are performed in a second droplet. For example, the tagmentation steps of FIG. 4A are performed during the cell barcoding 170 step shown in FIG. 1B. Thus, in such embodiments, tagmentation of the genomic DNA occurs in the presence of nucleic acid amplification reagents that are used to perform nucleic acid amplification.

Top middle panel 400A depicts the double stranded free genomic DNA 340 (e.g., free gDNA 340 shown in FIG. 3C or free gDNA 340 shown in FIG. 3F). Middle panel 400B shows the formation of a transposome synaptic complex bound to the free genomic DNA 340. Here, the transposome synaptic complex includes two transposases 410. The two transposases 410 form a dimeric complex. Each transposase 410 can be linked to one or more adaptor sequences 420 (e.g., 420A, 420B, 420C, or 420D). Here, the adaptor sequences 420 can be designed such that their sequences are complementary to primer sequences that enable nucleic acid amplification and/or incorporation of barcode sequences.

Bottom middle panel 400C depicts genomic DNA fragments 405 (e.g., 405A and 405B) derived from the free genomic DNA 340. Here, the genomic DNA fragments 405 have now been cleaved by the transposases 410. In various embodiments, the transposases 410 may cleave the genomic DNA 340 such that the DNA fragments 405 have staggered cuts. Therefore, the DNA fragments 405 may have one or more gaps, such as gap 430A and 430B, that arise due to the staggered cut. The adaptor sequences 420 (e.g., 420A, 420B, 420C, and/or 420D) are inserted into the genomic DNA fragment 405. As shown in bottom middle panel 400C, the adaptor sequences 420 are inserted at the ends of the DNA fragments 405. Here, the gaps 430A and 430B remain within the DNA fragments 405.

Nucleic acid extension is performed by enzymes to fill in gaps in the DNA fragments 405 as a result of the cleavage and/or insertion of the adaptor sequences 420. In various embodiments, the enzymes are DNA polymerases. As shown in bottom panel 400D, the gaps 430A and 430B that were present in the DNA fragments 405 in panel 400C are now filled in. Additionally, nucleic acid extension if further performed on one or more of the adapter sequences. For example, adaptor sequence 420A in panel 400C is extended to sequence 440, which is complementary to adaptor sequence 420B. As another example, adaptor sequence 420D is extended to sequence 445, which is complementary to adaptor sequence 420C. Following nucleic acid extension, the nucleic acid product shown in panel 400D is referred to herein as tagmented genomic DNA 480.

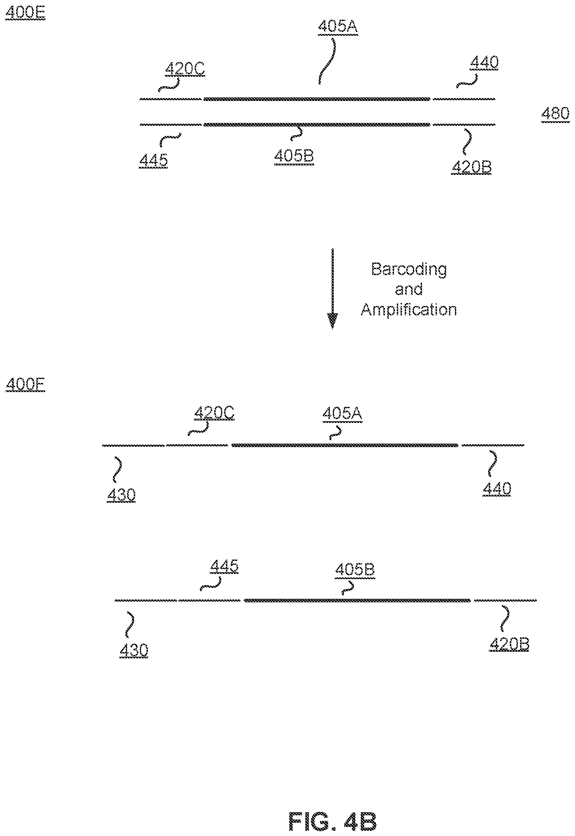

FIG. 4B depicts the amplification and barcoding of tagmented genomic DNA, in accordance with the embodiment shown in FIG. 4A. Here, top panel 400E shows the tagmented genomic DNA 480 generated following nucleic acid extension in panel 400D in FIG. 4A. The tagmented genomic DNA 480 undergoes barcoding and nucleic acid amplification.

In various embodiments, DNA fragment 405A and DNA fragment 405B and separately primed using one or more primers, and nucleic acid amplification can occur starting at the primer locations. In various embodiments, DNA fragment 405A is primed using a primer pair (e.g., forward primer and reverse primer pair) and DNA fragment 405B is primed using a primer pair (e.g., forward primer and reverse primer pair). Thus, DNA fragment 405A and DNA fragment 405B can be separately amplified.

In various embodiments, for amplification of DNA fragment 405A, a reverse primer can hybridize with sequence 440 and a forward primer can hybridize with sequence 420C. In various embodiments, the forward primer can further include a barcode sequence, such a barcode sequence provided by a barcoded bead, which is described in further detail below. Thus, over subsequent nucleic acid amplification cycles, the barcode sequence can be incorporated into the DNA amplicon. For example, as shown in bottom panel 400F, a DNA amplicon can include DNA fragment 405A, sequence 440, adaptor sequence 420C, and barcode sequence 430.

In various embodiments, for amplification of DNA fragment 405B, a reverse primer can hybridize with sequence 420B and a forward primer can hybridize with sequence 430. In various embodiments, the forward primer can further include a barcode sequence, such a barcode sequence provided by a barcoded bead, which is described in further detail below. Thus, over subsequent nucleic acid amplification cycles, the barcode sequence can be incorporated into the DNA amplicon. For example, as shown in bottom panel 400F, a DNA amplicon can include DNA fragment 405B, sequence 445, adaptor sequence 420B, and barcode sequence 430.

Thus, DNA amplicons derived from the tagmented genomic DNA 480 include barcode sequences 430, thereby enabling the subsequent determination that these amplicons originate from a single cell. These DNA amplicons shown in bottom panel 400F can undergo sequencing, such as whole genome sequencing, and further analyzed to characterize single-cells.

### Example Protocols for Genomic DNA release, Tagmentation, and Amplification

Embodiments described herein refer to protocols for release of genomic DNA from chromatin, tagmentation of free genomic DNA, and nucleic acid amplification. In various embodiments, release of genomic DNA and tagmentation of free genomic DNA occur within the same droplet. In such embodiments, protocols can involve exposing the droplet to different temperature ranges to enable the release of genomic DNA and tagmentation of free genomic DNA. In various embodiments, tagmentation of free genomic DNA and nucleic acid amplification occur within the same droplet. In such embodiments, protocols can involve exposing the droplet to different temperature ranges to enable tagmentation of free genomic DNA and nucleic acid amplification.

### Protocol for Genomic DNA Release

In various embodiments, genomic DNA is released from chromatin through the exposure of the chromatin to an enzyme. In various embodiments, the enzyme is a temperature sensitive enzyme. For example, the enzyme can be a protease that is active in a first temperature range, but is inactive in a different temperature range. For example, the protease can be proteinase K. In various embodiments, a droplet containing the protease and chromatin is exposed to a first temperature to activate the protease such that the protease can release genomic DNA from the chromatin. In various embodiments, the droplet is exposed to a first temperature between 30°C and 60°C. In various embodiments, the droplet is exposed to a first temperature between 35°C and 55°C. In various embodiments, the droplet is exposed to a first temperature between 40°C and 55°C. In various embodiments, the droplet is exposed to a first temperature between 45°C and 54°C. In various embodiments, the droplet is exposed to a first temperature between 48°C and 52°C. In particular embodiments, the droplet is exposed to a first temperature of about 50°C. In various embodiments, the droplet is exposed to the first temperature for between 5 minutes and 100 minutes. In various embodiments, the droplet is exposed to the first temperature for between 10 minutes and 95 minutes. In various embodiments, the droplet is exposed to the first temperature for between 20 minutes and 90 minutes. In various embodiments, the droplet is exposed to the first temperature for between 30 minutes and 80 minutes. In various embodiments, the droplet is exposed to the first temperature for between 40 minutes and 80 minutes. In various embodiments, the droplet is exposed to the first temperature for between 50 minutes and 70 minutes. In various embodiments, the droplet is exposed to the first temperature for between 55 minutes and 65 minutes. In various embodiments, the droplet is exposed to the first temperature for between 57 minutes and 63 minutes. In various embodiments, the droplet is exposed to the first temperature for about 60 minutes.

In various, a droplet containing the protease and chromatin is exposed to a temperature higher than the first temperature to inactivate the protease. In various embodiments, the droplet is exposed to a higher temperature between 70°C and 90°C. In various embodiments, the droplet is exposed to a higher temperature between 75°C and 85°C. In various embodiments, the droplet is exposed to a higher temperature between 78°C and 82°C. In various embodiments, the droplet is exposed to a higher temperature of about 80°C. In various embodiments, the droplet is exposed to a higher temperature of about 90°C. In various embodiments, the droplet is exposed to the higher temperature for between 1 minute and 20 minutes. In various embodiments, the droplet is exposed to the higher temperature for between 5 minutes and 15 minutes. In various embodiments, the droplet is exposed to the higher temperature for between 8 minutes and 12 minutes. In various embodiments, the droplet is exposed to the higher temperature for about 10 minutes. In various embodiments, the droplet is exposed to the higher temperature for between 30 minutes and 60 minutes. In various embodiments, the droplet is exposed to the higher temperature for about 30 minutes. In various embodiments, the droplet is exposed to the higher temperature for about 45 minutes. In various embodiments, the droplet is exposed to the higher temperature for about 60 minutes.

In particular embodiments, the protocol for genomic DNA release involves exposing the droplet to a first temperature between 40°C and 60°C for between 50 minutes and 70 minutes, then exposing the droplet to a second temperature between 70°C and 90°C for between 1 and 20 minutes.

### Protocol for Tagmentation

In various embodiments, genomic DNA undergoes tagmentation using transposases and an enzyme for performing nucleic acid extension, such as DNA polymerase or reverse transcriptase. In various embodiments, the transposases are active (e.g., able to cleave genomic DNA) in a first temperature range and the enzyme for performing nucleic acid extension is active (e.g., able to extend nucleic acids) in a second temperature range. In various embodiments, the transposase and the enzyme for performing nucleic acid extension are active in different temperature ranges. In various embodiments, the transposase and the enzyme for performing nucleic acid extension are active in overlapping temperature ranges. In various embodiments, the transposase is active in a temperature range between 35°C and 55°C. In various embodiments, the transposase is active in a temperature range between 35°C and 50°C. In various embodiments, the transposase is active in a temperature range between 40°C and 45°C.

In various embodiments, the enzyme for performing nucleic acid extension is active in a temperature range between 60°C and 80°C. In various embodiments, the enzyme for performing nucleic acid extension is active in a temperature range between 62°C and 78°C. In various embodiments, the enzyme for performing nucleic acid extension is active in a temperature range between 65°C and 75°C. In various embodiments, the enzyme for performing nucleic acid extension is active in a temperature range between 68°C and 72°C. In various embodiments, the enzyme for performing nucleic acid extension is active in a temperature range between 62°C and 70°C. In such embodiments, the enzyme for performing nucleic acid extension is active in a temperature range between 65°C and 68°C. In various embodiments, the enzyme for performing nucleic acid extension is a hotstart DNA polymerase.

In various embodiments, the enzyme for performing nucleic acid extension is active in a temperature range between 35°C and 65°C. In various embodiments, the enzyme for performing nucleic acid extension is active in a temperature range between 40°C and 60°C. In various embodiments, the enzyme for performing nucleic acid extension is active in a temperature range between 45°C and 55°C. In various embodiments, the enzyme for performing nucleic acid extension is active in a temperature range between 35°C and 45°C. In various embodiments, the enzyme for performing nucleic acid extension is active in a temperature range between 55°C and 65°C. In such embodiments, the enzyme for performing nucleic acid extension is an isothermal DNA polymerase. Examples of isothermal DNA polymerase include *Bacillus stearothermophilus* (Bst) DNA polymerase, such as Bst 2.0 or Bst 3.0 DNA polymerase.

In various embodiments, the enzyme for performing nucleic acid extension is active in a temperature range between 40°C and 50°C. In various embodiments, the enzyme for performing nucleic acid extension is active in a temperature range between 42°C and 48°C. In various embodiments, the enzyme for performing nucleic acid extension is active in a temperature range between 44°C and 46°C. In such embodiments, the enzyme for performing nucleic acid extension is a reverse transcriptase.

In various embodiments, the tagmentation protocol involves exposing a droplet to at least a first temperature, a second temperature, and a third temperature to enable cleavage of genomic DNA, insertion of adaptor sequences, and nucleic acid extension. Generally, the first temperature is lower than the second temperature, which is lower than the third temperature.

In various embodiments, the droplet is exposed to a first temperature between 35°C and 55°C. In various embodiments, the droplet is exposed to a first temperature between 35°C and 50°C. In various embodiments, the droplet is exposed to a first temperature between 35°C and 45°C. In various embodiments, the droplet is exposed to a first temperature between 35°C and 40°C. In particular embodiments, the droplet is exposed to a first temperature of about 37°C. In various embodiments, the droplet is exposed to the first temperature for between 5 minutes and 120 minutes. In various embodiments, the droplet is exposed to the first temperature for between 10 minutes and 100 minutes. In various embodiments, the droplet is exposed to the first temperature for between 20 minutes and 80 minutes. In various embodiments, the droplet is exposed to the first temperature for between 25 minutes and 60 minutes. In various embodiments, the droplet is exposed to the first temperature for between 30 minutes and 50 minutes. In particular embodiments, the droplet is exposed to the first temperature for about 30 minutes. In particular embodiments, the droplet is exposed to the first temperature for about 40 minutes. In particular embodiments, the droplet is exposed to the first temperature for about 50 minutes. In particular embodiments, the droplet is exposed to the first temperature for about 60 minutes.

In particular embodiments, the droplet is exposed to a first temperature between 35°C and 40°C for between 30 minutes and 50 minutes. In particular embodiments, the droplet is exposed to a first temperature of about 37°C for about 30 minutes.

In various embodiments, the droplet is exposed to a second temperature between 40°C and 100°C. In various embodiments, the droplet is exposed to a second temperature between 50°C and 90°C. In various embodiments, the droplet is exposed to a second temperature between 60°C and 80°C. In various embodiments, the droplet is exposed to a second temperature between 45°C and 75°C. In various embodiments, the droplet is exposed to a second temperature between 50°C and 65°C. In various embodiments, the droplet is exposed to a second temperature between 50°C and 60°C. In various embodiments, the droplet is exposed to a second temperature between 50°C and 55°C. In various embodiments, the droplet is exposed to a second temperature between 60°C and 70°C. In various embodiments, the droplet is exposed to a second temperature between 65°C and 70°C. In particular embodiments, the droplet is exposed to a second temperature of about 50°C. In particular embodiments, the droplet is exposed to a second temperature of about 60°C. In particular embodiments, the droplet is exposed to a second temperature of about 65°C. In various embodiments, the droplet is exposed to the second temperature for between 1 minute and 20 minutes. In various embodiments, the droplet is exposed to the second temperature for between 1 minute and 15 minutes. In various embodiments, the droplet is exposed to the second temperature for between 1 minute and 10 minutes. In various embodiments, the droplet is exposed to the second temperature for between 3 minute and 6 minutes. In various embodiments, the droplet is exposed to the second temperature for about 5 minutes. In some embodiments, the droplet is exposed to the second temperature for between 40 minutes and 80 minutes. In various embodiments, the droplet is exposed to the second temperature for between 50 minutes and 70 minutes. In various embodiments, the droplet is exposed to the second temperature for between 55 minutes and 65 minutes. In various embodiments, the droplet is exposed to the second temperature for between 57 minutes and 63 minutes. In various embodiments, the droplet is exposed to the second temperature for about 60 minutes.

In particular embodiments, the droplet is exposed to a second temperature between 60°C and 70°C for between 1 minute and 10 minutes. In particular embodiments, the droplet is exposed to a second temperature between 60°C and 70°C for between 3 minutes and 6 minutes.

In various embodiments, the droplet is exposed to a third temperature between 68°C and 85°C. In various embodiments, the droplet is exposed to a third temperature between 70°C and 82°C. In various embodiments, the droplet is exposed to a third temperature between 70°C and 80°C. In various embodiments, the droplet is exposed to a third temperature between 72°C and 80°C. In various embodiments, the droplet is exposed to a third temperature between 74°C and 78°C. In particular embodiments, the droplet is exposed to a third temperature of about 72°C. In particular embodiments, the droplet is exposed to a third temperature of about 75°C. In particular embodiments, the droplet is exposed to a third temperature of about 80°C. In various embodiments, the droplet is exposed to the third temperature for between 1 minute and 20 minutes. In various embodiments, the droplet is exposed to the third temperature for between 3 minutes and 15 minutes. In various embodiments, the droplet is exposed to the third temperature for between 5 minutes and 12 minutes. In various embodiments, the droplet is exposed to the third temperature for between 8 minute and 12 minutes. In various embodiments, the droplet is exposed to the third temperature for about 10 minutes. In various embodiments, the droplet is exposed to the third temperature for between 1 minutes and 10 minutes. In various embodiments, the droplet is exposed to the third temperature for between 1 minutes and 5 minutes. In various embodiments, the droplet is exposed to the third temperature for between 2 minute and 4 minutes. In various embodiments, the droplet is exposed to the third temperature for about 3 minutes.

In particular embodiments, the droplet is exposed to a third temperature between 70°C and 80°C for between 2 minutes and 4 minutes.

In particular embodiments, the tagmentation protocol involves exposing a droplet to a first temperature of about 37°C for about 30 minutes, then exposing the droplet to a second temperature of about 65°C for about 5 minutes, and further exposing the droplet to a third temperature of about 72°C for about 3 minutes to enable cleavage of genomic DNA, insertion of adaptor sequences, and nucleic acid extension.

### Protocol for Genomic DNA Release and Tagmentation in a Droplet

In various embodiments, the release of genomic DNA from chromatin and tagmentation of genomic DNA are performed in a single droplet (e.g., first droplet). In various embodiments, genomic DNA is released prior to tagmentation. In various embodiments, genomic DNA release and tagmentation is performed simultaneously. For example, at a particular temperature, proteases interact with packaged genomic DNA for releasing the genomic DNA and transposases interact with accessible regions of the genomic DNA for cleaving and tagging. Here, as genomic DNA is released and more regions of the genomic DNA becomes accessible, transposases can interact with these additional accessible regions for cleavage and tagging. In various embodiments, tagmentation occurs, at least in part, prior to genomic release. For example, tagmentation can occur on accessible regions of genomic DNA (e.g., regions of genomic DNA that are not bound by histones and/or packaged). Then, genomic DNA can be subsequently released from chromatin packaging, thereby enabling access to additional regions of the genomic DNA. Thus, tagmentation can further occur on these additional regions of the genomic DNA that are now accessible.

In various embodiments, the protocol for performing genomic DNA release and tagmentation can be any of: the protocol for performing genomic DNA release, the protocol for tagmentation of genomic DNA, or a modified version of the protocol for tagmentation of genomic DNA.

In some embodiments, the protocol for performing genomic DNA release and tagmentation can be the protocol for performing genomic DNA release, as described above. For example, the protocol for performing genomic DNA release and tagmentation can involve exposing the droplet to a first temperature between 40°C and 60°C for between 50 minutes and 70 minutes, then exposing the droplet to a second temperature between 70°C and 90°C for between 1 and 20 minutes. As another example, the protocol for performing genomic DNA release and tagmentation can involve exposing the droplet to a first temperature of about 50°C for about 60 minutes, then exposing the droplet to a second temperature of about 80°C for about 10 minutes.

In some embodiments, the protocol for performing genomic DNA release and tagmentation can be the protocol for performing tagmentation of genomic DNA, as described above. For example, the protocol for performing genomic DNA release and tagmentation can involve exposing a droplet to a first temperature of about 37°C for about 30 minutes, then exposing the droplet to a second temperature of about 65°C for about 5 minutes, and further exposing the droplet to a third temperature of about 72°C for about 3 minutes to enable cleavage of genomic DNA, insertion of adaptor sequences, and nucleic acid extension.

In some embodiments, the protocol for performing genomic DNA release and tagmentation can be a modified version of the protocol for tagmentation of genomic DNA, as described above. In various embodiments, the combined gDNA release and tagmentation protocol involves exposing a droplet to at least a first temperature, a second temperature, a third temperature, and a fourth temperature to enable release of gDNA, cleavage of gDNA, insertion of adaptor sequences, and nucleic acid extension. Generally, the first temperature is lower than the second temperature, which is lower than the third temperature, which is lower than the fourth temperature.

In various embodiments, the droplet is exposed to a first temperature between 35°C and 55°C. In various embodiments, the droplet is exposed to a first temperature between 35°C and 50°C. In various embodiments, the droplet is exposed to a first temperature between 35°C and 45°C. In various embodiments, the droplet is exposed to a first temperature between 35°C and 40°C. In particular embodiments, the droplet is exposed to a first temperature of about 37°C. In various embodiments, the droplet is exposed to the first temperature for between 5 minutes and 120 minutes. In various embodiments, the droplet is exposed to the first temperature for between 10 minutes and 100 minutes. In various embodiments, the droplet is exposed to the first temperature for between 20 minutes and 80 minutes. In various embodiments, the droplet is exposed to the first temperature for between 25 minutes and 60 minutes. In various embodiments, the droplet is exposed to the first temperature for between 30 minutes and 50 minutes. In particular embodiments, the droplet is exposed to the first temperature for about 30 minutes.

In various embodiments, the droplet is exposed to a second temperature between 40°C and 70°C. In various embodiments, the droplet is exposed to a second temperature between 50°C and 65°C. In various embodiments, the droplet is exposed to a second temperature between 50°C and 60°C. In various embodiments, the droplet is exposed to a second temperature between 50°C and 55°C. In various embodiments, the droplet is exposed to a second temperature between 60°C and 70°C. In various embodiments, the droplet is exposed to a second temperature between 65°C and 70°C. In particular embodiments, the droplet is exposed to a second temperature of about 50°C. In particular embodiments, the droplet is exposed to a second temperature of about 60°C. In particular embodiments, the droplet is exposed to a second temperature of about 65°C. In various embodiments, the droplet is exposed to the second temperature for between 1 minute and 20 minutes. In various embodiments, the droplet is exposed to the second temperature for between 1 minute and 15 minutes. In various embodiments, the droplet is exposed to the second temperature for between 1 minute and 10 minutes. In various embodiments, the droplet is exposed to the second temperature for between 3 minute and 6 minutes. In various embodiments, the droplet is exposed to the second temperature for about 5 minutes.

In various embodiments, the droplet is exposed to a third temperature between 68°C and 85°C. In various embodiments, the droplet is exposed to a third temperature between 70°C and 82°C. In various embodiments, the droplet is exposed to a third temperature between 70°C and 80°C. In various embodiments, the droplet is exposed to a third temperature between 72°C and 80°C. In various embodiments, the droplet is exposed to a third temperature between 72°C and 78°C. In various embodiments, the droplet is exposed to a third temperature between 72°C and 75°C. In various embodiments, the droplet is exposed to a third temperature between 74°C and 78°C. In some embodiments, the droplet is exposed to the third temperature for between 40 minutes and 80 minutes. In various embodiments, the droplet is exposed to the third temperature for between 50 minutes and 70 minutes. In various embodiments, the droplet is exposed to the third temperature for between 55 minutes and 65 minutes. In various embodiments, the droplet is exposed to the third temperature for between 57 minutes and 63 minutes. In various embodiments, the droplet is exposed to the third temperature for about 60 minutes.

In various embodiments, the droplet is exposed to a fourth temperature between 80°C and 100°C. In various embodiments, the droplet is exposed to a fourth temperature between 85°C and 95°C. In various embodiments, the droplet is exposed to a fourth temperature between 90°C and 95°C. In various embodiments, the droplet is exposed to a fourth temperature between 90°C and 100°C. In various embodiments, the droplet is exposed to a fourth temperature between 92°C and 98°C. In various embodiments, the droplet is exposed to a fourth temperature between 94°C and 96°C. In particular embodiments, the droplet is exposed to a fourth temperature of about 95°C. In some embodiments, the droplet is exposed to the fourth temperature for between 1 minute and 40 minutes. In various embodiments, the droplet is exposed to the fourth temperature for between 10 minutes and 30 minutes. In various embodiments, the droplet is exposed to the fourth temperature for between 15 minutes and 25 minutes. In various embodiments, the droplet is exposed to the fourth temperature for between 18 minutes and 22 minutes. In various embodiments, the droplet is exposed to the fourth temperature for about 20 minutes.

In particular embodiments, the combined gDNA release and tagmentation protocol involves exposing a droplet to about 37°C for about 30 minutes, then exposing the droplet to about 65°C for about 5 minutes, further exposing the droplet to about 75°C for about 60 minutes, and further exposing the droplet to about 95°C for about 20 minutes to enable release of gDNA, cleavage of gDNA, insertion of adaptor sequences, and nucleic acid extension.

### Protocol for Nucleic Acid Amplification

In various embodiments, the nucleic amplification protocol can involve multiple cycles of denaturation, annealing, and nucleic acid extension. For example, the nucleic acid amplification protocol can involve exposing a droplet to a denaturation temperature between 90°C and 100°C (e.g., 98°C), followed by exposure of the droplet to an annealing temperature between 55°C and 65°C (e.g., 61°C), followed by exposure of the to an extension temperature between 65°C and 75°C (e.g., 72°C). In particular embodiments, the nucleic acid amplification protocol includes exposing the droplet to the following temperatures: 1) 98°C for 30 seconds, 2) 10 cycles of 98°C for 10 seconds followed by 72°C for 45 seconds, 3) 10 cycles of 98°C for 30 seconds followed by 61°C for 30 seconds and followed by 72°C for 45 seconds, and 3) 1 cycle of 72°C for 3 minutes.

In various embodiments, the nucleic amplification reaction can be an isothermal amplification reaction. In such embodiments, the nucleic acid amplification protocol can involve exposing the droplet to two temperatures. For example, the first temperature can be between 60°C and 70°C (e.g., 65°C). In various embodiments, the droplet is exposed to the first temperature for about 2 hours. The second temperature can be between 75°C and 85°C. In various embodiments, the droplet is exposed to the first temperature for about 30 minutes.

### Protocol for Tagmentation and Nucleic Acid Amplification in a Droplet

In various embodiments, tagmentation and nucleic acid amplification occur in a single droplet (e.g., a second droplet). In various embodiments, the combination tagmentation and amplification protocol involves combining the tagmentation protocol described above and the nucleic acid amplification protocol described above. In various embodiments, the combination tagmentation and amplification protocol involves first performing tagmentation (according to the tagmentation protocol describe above) and subsequently performing nucleic acid amplification (according to the nucleic acid amplification protocol described above).

In various embodiments, the tagmentation protocol involves exposing a droplet to at least a first temperature, a second temperature, and a third temperature to enable cleavage of genomic DNA, insertion of adaptor sequences, and nucleic acid extension. Generally, the first temperature is lower than the second temperature, which is lower than the third temperature. In particular embodiments, the droplet is exposed to a first temperature between 35°C and 40°C for between 30 minutes and 50 minutes. In particular embodiments, the droplet is exposed to a first temperature of about 37°C for about 30 minutes. In particular embodiments, the droplet is exposed to a second temperature between 60°C and 70°C for between 3 minutes and 6 minutes. In particular embodiments, the droplet is exposed to a second temperature of about 65°C for about 5 minutes. In particular embodiments, the droplet is exposed to a third temperature between 70°C and 80°C for between 2 minutes and 4 minutes. In particular embodiments, the droplet is exposed to a third temperature of about 72°C for about 3 minutes. In particular embodiments, the tagmentation protocol involves exposing a droplet to a first temperature of about 37°C for about 30 minutes, then exposing the droplet to a second temperature of about 65°C for about 5 minutes, and further exposing the droplet to a third temperature of about 72°C for about 3 minutes to enable cleavage of genomic DNA, insertion of adaptor sequences, and nucleic acid extension.

After the tagmentation protocol, the tagmented genomic DNA (e.g., DNA fragments with adaptor sequences) undergo nucleic acid amplification. In some embodiments, the nucleic acid amplification protocol involves multiple cycles of denaturation, annealing, and nucleic acid extension, as is described above. In some embodiments, the nucleic acid amplification protocol involves an isothermal amplification reaction, as is described above.

### Barcodes and Barcoded Beads

Embodiments of the invention involve providing one or more barcode sequences for labeling analytes of a single cell during step 170 shown in FIG. 1B and/or for labeling tagmented genomic DNA 480 shown in FIG. 4B. The one or more barcode sequences are encapsulated in an emulsion with a cell lysate derived from a single cell. As such, the one or more barcodes label analytes, such as tagmented genomic DNA, of the cell, thereby enabling the subsequent determination that sequence reads derived from the analytes originated from the cell.

In various embodiments, a plurality of barcodes are added to an emulsion with a cell lysate. In various embodiments, the plurality of barcodes added to an emulsion includes at least 10², at least 10³, at least 10⁴, at least 10⁵, at least 10⁵, at least 10⁶, at least 10⁷, or at least 10⁸ barcodes. In various embodiments, the plurality of barcodes added to an emulsion have the same barcode sequence. In various embodiments, the plurality of barcodes added to an emulsion comprise a 'unique identification sequence' (UMI). A UMI is a nucleic acid having a sequence which can be used to identify and/or distinguish one or more first molecules to which the UMI is conjugated from one or more second molecules. UMIs are typically short, e.g., about 5 to 20 bases in length, and may be conjugated to one or more target molecules of interest or amplification products thereof. UMIs may be single or double stranded. In some embodiments, both a barcode sequence and a UMI are incorporated into a barcode. Generally, a UMI is used to distinguish between molecules of a similar type within a population or group, whereas a barcode sequence is used to distinguish between populations or groups of molecules that are derived from different cells. Thus, a UMI can be used to count or quantify numbers of particular molecules (e.g., quantify number of RNA transcripts). In some embodiments, where both a UMI and a barcode sequence are utilized, the UMI is shorter in sequence length than the barcode sequence. The use of barcodes is further described in US Patent Publication Application No. US 2018/0216160 A1.

In some embodiments, the barcodes are single-stranded barcodes. Single-stranded barcodes can be generated using a number of techniques. For example, they can be generated by obtaining a plurality of DNA barcode molecules in which the sequences of the different molecules are at least partially different. These molecules can then be amplified so as to produce single stranded copies using, for instance, asymmetric PCR. Alternatively, the barcode molecules can be circularized and then subjected to rolling circle amplification. This will yield a product molecule in which the original DNA barcoded is concatenated numerous times as a single long molecule.

In some embodiments, circular barcode DNA containing a barcode sequence flanked by any number of constant sequences can be obtained by circularizing linear DNA. Primers that anneal to any constant sequence can initiate rolling circle amplification by the use of a strand displacing polymerase (such as Phi29 polymerase), generating long linear concatemers of barcode DNA.

In various embodiments, barcodes can be linked to a primer sequence that enables the barcode to label a target nucleic acid. In one embodiment, the barcode is linked to a forward primer sequence. In various embodiments, the forward primer sequence is a gene specific primer that hybridizes with a forward target of a nucleic acid. In various embodiments, the forward primer sequence is a constant region, such as a PCR handle, that hybridizes with a complementary sequence attached to a gene specific primer. The complementary sequence attached to a gene specific primer can be provided in the reaction mixture (e.g., reaction mixture 140 in FIG. 1B). Including a constant forward primer sequence on barcodes may be preferable as the barcodes can have the same forward primer and need not be individually designed to be linked to gene specific forward primers.

In various embodiments, barcodes can releasably attached to a support structure, such as a bead. Therefore, a single bead with multiple copies of barcodes can be partitioned into an emulsion with a cell lysate, thereby enabling labeling of analytes of the cell lysate with the barcodes of the bead. Example beads include solid beads (e.g., silica beads), polymeric beads, or hydrogel beads (e.g., polyacrylamide, agarose, or alginate beads). Beads can be synthesized using a variety of techniques. For example, using a mix-split technique, beads with many copies of the same, random barcode sequence can be synthesized. This can be accomplished by, for example, creating a plurality of beads including sites on which DNA can be synthesized. The beads can be divided into four collections and each mixed with a buffer that will add a base to it, such as an A, T, G, or C. By dividing the population into four subpopulations, each subpopulation can have one of the bases added to its surface. This reaction can be accomplished in such a way that only a single base is added and no further bases are added. The beads from all four subpopulations can be combined and mixed together, and divided into four populations a second time. In this division step, the beads from the previous four populations may be mixed together randomly. They can then be added to the four different solutions, adding another, random base on the surface of each bead. This process can be repeated to generate sequences on the surface of the bead of a length approximately equal to the number of times that the population is split and mixed. If this was done 10 times, for example, the result would be a population of beads in which each bead has many copies of the same random 10-base sequence synthesized on its surface. The sequence on each bead would be determined by the particular sequence of reactors it ended up in through each mix-split cycle. Additional details of example beads and their synthesis is described in International Application No. PCT/US2016/016444.

### Reagents

Embodiments described herein include the encapsulation of a cell with reagents within an emulsion. In various embodiments, the reagents interact with the encapsulated cell under conditions in which the cell is lysed, thereby releasing target analytes of the cell. The reagents can further interact with target analytes to prepare for subsequent barcoding and/or amplification.

In various embodiments, the reagents include one or more lysing agents that cause the cell to lyse. Examples of lysing agents include detergents such as Triton X-100, Nonidet P-40 (NP40) as well as cytotoxins. In various embodiments include 0.01%, 0.05%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, or 5.0%, 5.5%, 6%, 6.5%, 7%, 7.5%, 8%, 8.5%, 9%, 9.5%, or 10% NP40 (v/v). In various embodiments, the reagents include 1% NP40. In various embodiments, the reagents include 5% NP40. In particular embodiments, the reagents include 10% NP40.

In various embodiments, the reagents encapsulated with the cell include ddNTPs, inhibitors such as ribonuclease inhibitor, and stabilization agents such as dithothreitol (DTT). In various embodiments, the reagents further include proteases that assist in the lysing of the cell and/or accessing of genomic DNA. In various embodiments, proteases in the reagents can include any of proteinase K, pepsin, protease-subtilisin Carlsberg, protease type X-bacillus thermoproteolyticus, or protease type XIII-aspergillus Saitoi. In various embodiments, the reagents include deoxyribonucleotide triphosphate (dNTP) reagents including deoxyadenosine triphosphate, deoxycytosine triphosphate, deoxyguanine triphosphate, and deoxythymidine triphosphate.

In various embodiments, the reagents include agents that interact with target analytes that are released from a single cell. For example, the reagents include reverse transcriptase which reverse transcribes mRNA transcripts released from the cell to generate corresponding cDNA. As another example, the reagents include primers that hybridize with mRNA transcripts, thereby enabling the reverse transcription reaction to occur.

In various embodiments, the reagents include agents that enable tagmentation of genomic DNA. In such embodiments, tagmentation occurs in the same droplet as cell lysis and genomic DNA release. For example, the reagents can include transposases for cleaving genomic DNA. In various embodiments, the transposases include a MuA transposase or a Tn5 transposase (or mutated transposase Tn5). Examples of transposase Tn5 include Illumina Tagment DNA Enzyme (Illumina Catalog Numbers 20034197 or 20034198) and Nextera Tn5 Transposase, Illumina Cat #FC-121-1030.

In various embodiments, the reagents include an enzyme for performing nucleic acid extension on DNA fragments resulting from cleavage of genomic DNA. In various embodiments, the enzyme is a reverse transcriptase. In various embodiments, the enzyme is a DNA polymerase. Examples of DNA polymerase include HotStart polymerase (e.g., HotStarTaq DNA polymerase from Qiagen or Q5^{®} High Fidelity DNA polymerase from New England Biolabs) and isothermal DNA polymerases (e.g., *Bacillus stearothermophilus* (Bst) DNA polymerase, such as Bst 2.0 or Bst 3.0 DNA polymerase).

### Reaction Mixture

As described herein, a reaction mixture is provided into an emulsion with a cell lysate (e.g., see cell barcoding step 170 in FIG. 1B). Generally, the reaction mixture includes reactants sufficient for performing a reaction, such as nucleic acid amplification, on analytes of the cell lysate.

In various embodiments, the reaction mixture includes primers that are capable of acting as a point of initiation of synthesis along a complementary strand when placed under conditions in which synthesis of a primer extension product which is complementary to a nucleic acid strand is catalyzed. In various embodiments, the reaction mixture includes the four different deoxyribonucleoside triphosphates (adenosine, guanine, cytosine, and thymine). In various embodiments, the reaction mixture includes enzymes for nucleic acid amplification. Examples of enzymes for nucleic acid amplification include DNA polymerase, thermostable polymerases for thermal cycled amplification, or polymerases for multiple-displacement amplification for isothermal amplification. Other, less common forms of amplification may also be applied, such as amplification using DNA- dependent RNA polymerases to create multiple copies of RNA from the original DNA target which themselves can be converted back into DNA, resulting in, in essence, amplification of the target. Living organisms can also be used to amplify the target by, for example, transforming the targets into the organism which can then be allowed or induced to copy the targets with or without replication of the organisms.

In various embodiments, the reagents include deoxyribonucleotide triphosphate (dNTP) reagents including deoxyadenosine triphosphate, deoxycytosine triphosphate, deoxyguanine triphosphate, and deoxythymidine triphosphate. The extent of nucleic amplification can be controlled by modulating the concentration of the reactants in the reaction mixture. In some instances, this is useful for fine tuning of the reactions in which the amplified products are used.

In various embodiments, the reaction mixture include agents that enable tagmentation of genomic DNA. In such embodiments, tagmentation occurs in the same droplet as cell barcoding and nucleic acid amplification. For example, the reaction mixture can include transposases for cleaving genomic DNA. In various embodiments, the transposases include a MuA transposase or a Tn5 transposase (or mutated transposase Tn5). Examples of transposase Tn5 include Illumina Tagment DNA Enzyme (Illumina Catalog Numbers 20034197 or 20034198) and Nextera Tn5 Transposase, Illumina Cat #FC-121-1030.

In various embodiments, the reaction mixture includes an enzyme for performing nucleic acid extension on genomic DNA fragments resulting from cleavage of genomic DNA. In various embodiments, the enzyme is a DNA polymerase. Examples of DNA polymerase include HotStart polymerase (e.g., HotStarTaq DNA polymerase from Qiagen or Q5^{®} High Fidelity DNA polymerase from New England Biolabs) and isothermal DNA polymerases (e.g., *Bacillus stearothermophilus* (Bst) DNA polymerase, such as Bst 2.0 or Bst 3.0 DNA polymerase).

### Primers

Embodiments of the invention described herein use primers to conduct the single-cell analysis. For example, primers are implemented during the workflow process shown in FIG. 1B. Primers can be used to prime (e.g., hybridize) with specific sequences of nucleic acids of interest, such that the nucleic acids of interest can be processed (e.g., reverse transcribed, barcoded, and/or amplified). Additionally, primers enable the identification of target regions following sequencing.

In various embodiments, primers described herein are between 5 and 50 nucleobases in length. In various embodiments, primers described herein are between 7 and 45 nucleobases in length. In various embodiments, primers described herein are between 10 and 40 nucleobases in length. In various embodiments, primers described herein are between 12 and 35 nucleobases in length. In various embodiments, primers described herein are between 15 and 32 nucleobases in length. In various embodiments, primers described herein are between 18 and 30 nucleobases in length. In various embodiments, primers described herein are between 18 and 25 nucleobases in length.

Referring again to FIG. 1B, in various embodiments, primers can be included in the reagents 120 that are encapsulated with the cell 110. In various embodiments, primers included in the reagents are useful for priming RNA transcripts and enabling reverse transcription of the RNA transcripts. In various embodiments, primers in the reagents 120 can include RNA primers for priming RNA and/or for priming genomic DNA.

In various embodiments, primers can be included in the reaction mixture 140 that is encapsulated with the cell lysate 130. In various embodiments, primers included in the reaction mixture are useful for priming nucleic acids (e.g., cDNA, gDNA, and/or amplicons of cDNA/gDNA) and enabling nucleic acid amplification of the nucleic acids. Such primers in the reaction mixture 140 can include cDNA primers for priming cDNA that have been reverse transcribed from RNA and/or DNA primers for priming genomic DNA and/or for priming products that have been generated from the genomic DNA. In various embodiments, primers of the reagents and primers of the reaction mixture form primer sets (e.g., forward primer and reverse primer) for a region of interest on a nucleic acid. In various embodiments, primers can be included in or linked with a barcode 145 that is encapsulated with the cell lysate 130. Further description and examples of primers that are used in a single-cell analysis workflow process is described in US Application Publication No. US 2020/0232011 A1.

In various embodiments, the number of primers in any of the reagents, the reaction mixture, or with barcodes may range from about 1 to about 500 or more, e.g., about 2 to 100 primers, about 2 to 10 primers, about 10 to 20 primers, about 20 to 30 primers, about 30 to 40 primers, about 40 to 50 primers, about 50 to 60 primers, about 60 to 70 primers, about 70 to 80 primers, about 80 to 90 primers, about 90 to 100 primers, about 100 to 150 primers, about 150 to 200 primers, about 200 to 250 primers, about 250 to 300 primers, about 300 to 350 primers, about 350 to 400 primers, about 400 to 450 primers, about 450 to 500 primers, or about 500 primers or more.

In various embodiments, primers in the reagents or reaction mixture are designed for whole genome sequencing. Specifically, primers are designed to prime adaptor sequences of tagmented genomic DNA. For example, referring against to top panel 400E in FIG. 4B, primers can hybridize with any of sequences 420B, 420C, 440, or 445, thereby enabling nucleic acid extension off of the primer. Thus, given the presence of tagmented genomic DNA that span the whole genome, the primers enable whole genome nucleic acid amplification.

In various embodiments, primers in the reagents or reaction mixture are universal primers. Example universal primers include primers including at least 3 consecutive deoxythymidine nucleobases (e.g., oligo dT primer). In various embodiments, primers in the reagents are reverse primers. In particular embodiments, primers in the reagents are only reverse primers and do not include forward primers. In various embodiments, for targeted nucleic acid (e.g., targeted DNA or targeted RNA) sequencing, primers in the reaction mixture (e.g., reaction mixture 140 in FIG. 1B) include forward primers that are complementary to a forward target on a nucleic acid of interest (e.g., RNA or gDNA). In particular embodiments, the reaction mixture includes forward primers that are complementary to a forward target on a cDNA strand (generated from a RNA transcript) and further includes forward primers that are complementary to a forward target on gDNA. In various embodiments, primers in the reaction mixture are gene-specific primers that target a forward target of a gene of interest.

For whole transcriptome RNA sequencing, in various embodiments, the primers of the reagents (e.g., reagents 120 in FIG. 1B) can include a random primer sequence. In various embodiments, the random primer hybridizes with a sequence of reverse transcribed cDNA, thereby enabling priming off of the cDNA. In various embodiments, the reagents 120 includes various different random primers that enables priming off of all or a majority of cDNA generated from mRNA transcripts across the transcriptome. This enables the processing and analysis of mRNA transcripts across the whole transcriptome. In various embodiments, a random primer comprises a sequence of 5 nucleobases. In various embodiments, a random primer comprises a sequence of 6 nucleobases. In various embodiments, a random primer comprises a sequence of 9 nucleobases. In various embodiments, a random primer comprises a sequence of at least 5 nucleobases. In various embodiments, a random primer comprises a sequence of at least 6 nucleobases. In various embodiments, a random primer comprises a sequence of at least 9 nucleobases. In various embodiments, a random primer comprises a sequence of at least 6 nucleobases, at least 7 nucleobases, at least 8 nucleobases, at least 9 nucleobases, at least 10 nucleobases, at least 11 nucleobases, at least 12 nucleobases, at least 13 nucleobases, at least 14 nucleobases, at least 15 nucleobases, at least 16 nucleobases, at least 17 nucleobases, at least 18 nucleobases, at least 19 nucleobases, at least 20 nucleobases, at least 21 nucleobases, at least 22 nucleobases, at least 23 nucleobases, at least 24 nucleobases, at least 25 nucleobases, at least 26 nucleobases, at least 27 nucleobases, at least 28 nucleobases, at least 29 nucleobases, at least 30 nucleobases, at least 31 nucleobases, at least 32 nucleobases, at least 33 nucleobases, at least 34 nucleobases, or at least 35 nucleobases.

In various embodiments, a random primer includes one or more ribonucleotide nucleobases. In some embodiments, the random primer 624 include one ribonucleotide nucleobase on the 3' end. In some embodiments, the random primer 624 includes two ribonucleotide nucleobases on the 3' end. In some embodiments, the random primer 624 includes three, four, five, six, seven, eight, nine, or ten ribonucleotide nucleobases on the 3' end. The presence of ribonucleotide primers on the 3' end of the random primer ensures that the random primer enables extension only on cDNA and not on RNA.

In various embodiments, the reagents include a reverse primer that is complementary to a portion of mRNA transcripts. In various embodiments, the reverse primer is a universal primer, such as an oligo dT primer that hybridizes with the poly A tail of messenger RNA transcripts. Therefore, the reverse primer hybridizes with a portion of mRNA transcripts and enables generation of cDNA strands through reverse transcription of the mRNA transcripts.

In various embodiments, for whole transcriptome RNA sequencing, the primers of the reaction mixture (e.g., reaction mixture 140 in FIG. 1B) include constant forward primers and constant reverse primers. The constant forward primers hybridize with the random forward primer that enabled priming off the cDNA. The constant reverse primers hybridize with a sequence of the reverse constant region, such as a PCR handle, that previously enabled reverse transcription of the mRNA transcript.

In various embodiments, primers included in the reagents (e.g., reagents 120 in FIG. 1B) or the reaction mixture (e.g., reaction mixture 140 in FIG. 1B) include additional sequences. Such additional sequences may have functional purposes. For example, a primer may include a read sequence for sequencing purposes. As another example, a primer may include a constant region. Generally, the constant region of a primer can hybridize with a complementary constant region on another nucleic acid sequence for incorporation of the nucleic acid sequence during nucleic acid amplification. For example, the constant region of a primer can be complementary to a complementary constant region of a barcode sequence. Thus, during nucleic acid amplification, the barcode sequence is incorporated into generated amplicons.

In various embodiments, instead of the primers being included in the reaction mixture (e.g., reaction mixture 140 in FIG. 1B) such primers can be included or linked to a barcode (e.g., barcode 145 in FIG. 1B). In particular embodiments, the primers are linked to an end of the barcode and therefore, are available to hybridize with target sequences of nucleic acids in the cell lysate.

In various embodiments, primers of the reaction mixture, primers of the reagents, or primers of barcodes may be added to an emulsion in one step, or in more than one step. For instance, the primers may be added in two or more steps, three or more steps, four or more steps, or five or more steps. Regardless of whether the primers are added in one step or in more than one step, they may be added after the addition of a lysing agent, prior to the addition of a lysing agent, or concomitantly with the addition of a lysing agent. When added before or after the addition of a lysing agent, the primers of the reaction mixture may be added in a separate step from the addition of a lysing agent (e.g., as exemplified in the two step workflow process shown in FIG. 1B).

A primer set for the amplification of a target nucleic acid typically includes a forward primer and a reverse primer that are complementary to a target nucleic acid or the complement thereof. In some embodiments, amplification can be performed using multiple target-specific primer pairs in a single amplification reaction, wherein each primer pair includes a forward target-specific primer and a reverse target-specific primer, where each includes at least one sequence that substantially complementary or substantially identical to a corresponding target sequence in the sample, and each primer pair having a different corresponding target sequence. Accordingly, certain methods herein are used to detect or identify multiple target sequences from a single cell sample.

### Example System and/or Computer Embodiments

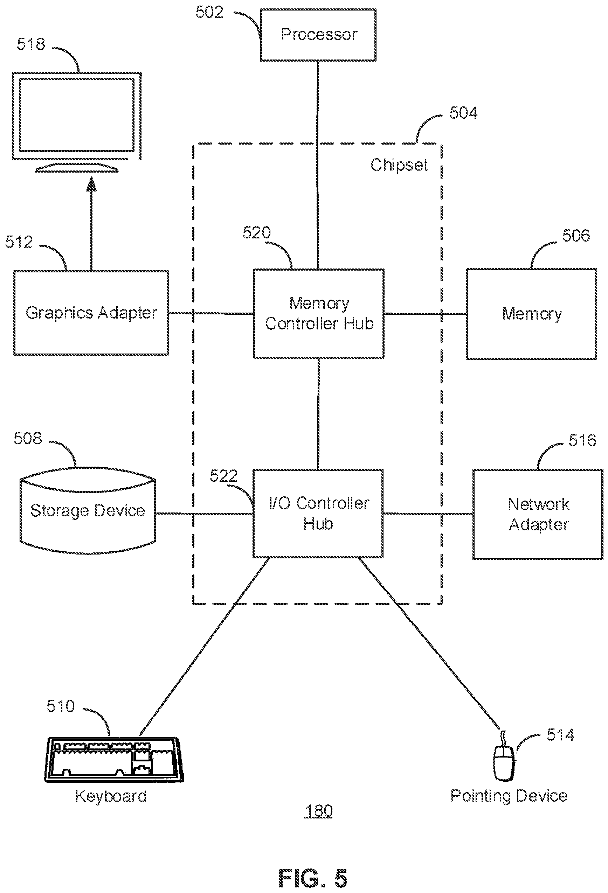

FIG. 5 depicts an example computing device (e.g., computing device 180 shown in FIG. 1A) for implementing system and methods described in reference to FIGs. 1-4B. For example, the example computing device 180 is configured to perform the *in silico* steps of read alignment 215 and/or characterization 220. Examples of a computing device can include a personal computer, desktop computer laptop, server computer, a computing node within a cluster, message processors, hand-held devices, multi-processor systems, microprocessor-based or programmable consumer electronics, network PCs, minicomputers, mainframe computers, mobile telephones, PDAs, tablets, pagers, routers, switches, and the like.

In some embodiments, the computing device 180 includes at least one processor 502 coupled to a chipset 504. The chipset 504 includes a memory controller hub 520 and an input/output (I/O) controller hub 522. A memory 506 and a graphics adapter 512 are coupled to the memory controller hub 520, and a display 518 is coupled to the graphics adapter 512. A storage device 508, an input interface 514, and network adapter 516 are coupled to the I/O controller hub 522. Other embodiments of the computing device 180 have different architectures.

The storage device 508 is a non-transitory computer-readable storage medium such as a hard drive, compact disk read-only memory (CD-ROM), DVD, or a solid-state memory device. The memory 506 holds instructions and data used by the processor 502. The input interface 514 is a touch-screen interface, a mouse, track ball, or other type of input interface, a keyboard, or some combination thereof, and is used to input data into the computing device 180. In some embodiments, the computing device 180 may be configured to receive input (e.g., commands) from the input interface 514 via gestures from the user. The graphics adapter 512 displays images and other information on the display 518. For example, the display 518 can show metrics pertaining to the generated libraries (e.g., DNA or RNA libraries) and/or any characterization of single cells. The network adapter 516 couples the computing device 180 to one or more computer networks.

The computing device 180 is adapted to execute computer program modules for providing functionality described herein. As used herein, the term "module" refers to computer program logic used to provide the specified functionality. Thus, a module can be implemented in hardware, firmware, and/or software. In one embodiment, program modules are stored on the storage device 508, loaded into the memory 506, and executed by the processor 502.

The types of computing devices 180 can vary from the embodiments described herein. For example, the computing device 180 can lack some of the components described above, such as graphics adapters 512, input interface 514, and displays 518. In some embodiments, a computing device 180 can include a processor 502 for executing instructions stored on a memory 506.

The methods of aligning sequence reads and characterizing libraries and/or cells can be implemented in hardware or software, or a combination of both. In one embodiment, a non-transitory machine-readable storage medium, such as one described above, is provided, the medium comprising a data storage material encoded with machine readable data which, when using a machine programmed with instructions for using said data, is capable of displaying any of the datasets and execution and results of this invention. Such data can be used for a variety of purposes, such as patient monitoring, treatment considerations, and the like. Embodiments of the methods described above can be implemented in computer programs executing on programmable computers, comprising a processor, a data storage system (including volatile and non-volatile memory and/or storage elements), a graphics adapter, an input interface, a network adapter, at least one input device, and at least one output device. A display is coupled to the graphics adapter. Program code is applied to input data to perform the functions described above and generate output information. The output information is applied to one or more output devices, in known fashion. The computer can be, for example, a personal computer, microcomputer, or workstation of conventional design.

Each program can be implemented in a high level procedural or object oriented programming language to communicate with a computer system. However, the programs can be implemented in assembly or machine language, if desired. In any case, the language can be a compiled or interpreted language. Each such computer program is preferably stored on a storage media or device (*e.g.,* ROM or magnetic diskette) readable by a general or special purpose programmable computer, for configuring and operating the computer when the storage media or device is read by the computer to perform the procedures described herein. The system can also be considered to be implemented as a computer-readable storage medium, configured with a computer program, where the storage medium so configured causes a computer to operate in a specific and predefined manner to perform the functions described herein.

The signature patterns and databases thereof can be provided in a variety of media to facilitate their use. "Media" refers to a manufacture that contains the signature pattern information of the present invention. The databases of the present invention can be recorded on computer readable media, *e.g.* any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable mediums can be used to create a manufacture comprising a recording of the present database information. "Recorded" refers to a process for storing information on computer readable medium, using any such methods as known in the art. Any convenient data storage structure can be chosen, based on the means used to access the stored information. A variety of data processor programs and formats can be used for storage, e.g. word processing text file, database format, etc.

### Additional Embodiments

Disclosed herein are methods, apparati, and systems for conducting whole genome sequencing amplification on microdroplet devices. As one example, the disclosure relates to methods, apparati, and systems for conducting whole genome sequencing amplification on a Tapestri^{™} device.

In an embodiment, the disclosure relates to a high throughput method, system and device to create whole genome libraries from single cells. The disclosed approach produces whole genome libraries where each fragment from a cell contains the same cell barcode. To produce these single cell whole genome libraries, the Tapestri^{™} system is used. With the two droplet system, the cells are encapsulated in the first droplet where they can be lysed and further exposed to protease to free the DNA from chromatin. With the DNA from each single cell still encapsulated in the droplets, the transposase reaction components can be introduced. With single droplet approaches, transposase reactions can be performed but the DNA is not released from the chromatin so this produces single cell ATAC-seq libraries, not single cell whole genome libraries.

On the Tapestri, in the second droplet, the transposase reaction can be combined with an amplification reaction along with the encapsulated cell lysate and barcoding bead. The transposase reaction could be performed at temperatures around 37C-50C. Using a hotstart polymerase that is active around 60-70C, the 3' end can be filled in before the DNA fragments are denatured. Many hotstart polymerases do possess some activity around 60-70C so in some embodiments, only one polymerase is needed for the fill in and amplification. Following the fill-in, whole genome amplification can be performed which includes incorporating the cell barcode sequence from the barcoding beads.

Once this emulsion is broken, whole genome fragments, each containing a cell barcode and with known sequences on both ends, are available for library preparation or further reactions. For example, at this stage, a probe based capture method could be used for targeted sequencing libraries. Alternatively, targeted sequencing libraries can be captured using a single gene specific primer or nested gene specific primers for anchored PCR. Additionally, these libraries could be used for methylome analysis by performing bisulfite treatment or enzymatic methyl-seq. The cell barcodes could have methylated bases incorporated if more diversity is needed after bisulfite conversion. Also, with the cell barcodes already attached, reduced restriction bisulfite sequencing can be performed.

### EXAMPLES

### Example 1: Single Cell Analysis Including Tagmentation in Second Droplet

GM24385 human cells were processed through the single cell workflow (e.g., Tapestri^{™}) described above in reference to FIG. 1B. In this example, cell lysis and release of genomic DNA occurred in the first droplet followed by tagmentation in the second droplet. Reagents included cell lysis buffer and proteinase K for releasing genomic DNA. Cell barcodes were additionally added to the second droplet for incorporation into amplicons during nucleic acid amplification.

Three different runs were performed with differing reaction mixture for performing tagmentation. The components of the reaction mixture and corresponding volumes for each of the three runs are shown below in Tables 1-3. The reaction mixture in all three runs included a ted-CapALL enzyme mix which further includes a Tn5 transposase for cleaving and inserting adapters into genomic DNA.

In particular, the reaction mixture of run 1 included a Q5 Master Mix including Q5 High Fidelity DNA polymerase. The reaction mixture of run 2 included a spike in of the Q5 HotStart DNA polymerase. The reaction mixture of run 3 included a spike in of Bst 2.0 DNA polymerase, which is an in silico designed homologue of *Bacillus stearothermophilus* DNA Polymerase I.

**Table 1: Volume of components in reaction mixture for run 1.**

| **Volume** | **Reaction Mixture** |
|---|---|
| 30 uL | ted-CapALL primer mix |
| 30 uL | ted-CapALL enzyme mix - Tn5 transposase |
| 90 uL | 2X Q5 MasterMix |

**Table 2: Volume of components in reaction mixture for run 2.**

| **Volume** | **Reaction Mixture** |
|---|---|
| 30 uL | ted-CapALL primer mix |
| 30 uL | ted-CapALL enzyme mix - kit |
| 3 uL | Q5 HS polymerase |
| 87 uL | dH2O |

**Table 3: Volume of components in reaction mixture for run 3.**

| **Volume** | **Reaction Mixture** |
|---|---|
| 30 uL | ted-CapALL primer mix |
| 30 uL | ted-CapALL enzyme mix - kit |
| 6 uL | Bst 2.0 |
| 84 uL | dH2O |

For each of runs 1, 2, and 3, droplets including the reaction mixture and cell lysate were processed according to the following preamplification cycling protocol: 1) 1 cycle of 37°C for 30 minutes, 2) 1 cycle of 65°C for 5 minutes, and 3) 1 cycle of 72°C for 3 minutes. Here, the preamplification cycling protocol enables tagmentation and extension to occur. Droplets were exposed to UV for 8 minutes.

For runs 1 and 2, the subsequent nucleic amplification cycling protocol is as follows: 1) 1 cycle of 98°C for 30 seconds, 2) 10 cycles of 98°C for 10 seconds followed by 72°C for 45 seconds, 3) 10 cycles of 98°C for 30 seconds followed by 61°C for 30 seconds, followed by 72°C for 45 seconds, 4) 72°C for 3 minutes, and 5) hold at 4°C. For run 3, the subsequent isothermal nucleic amplification cycling protocol is as follows: 1) 1 cycle of 65°C for 60 minutes.

Following amplification, amplicons were collected, sequenced, aligned, and distinguished according to presence of cell barcodes.

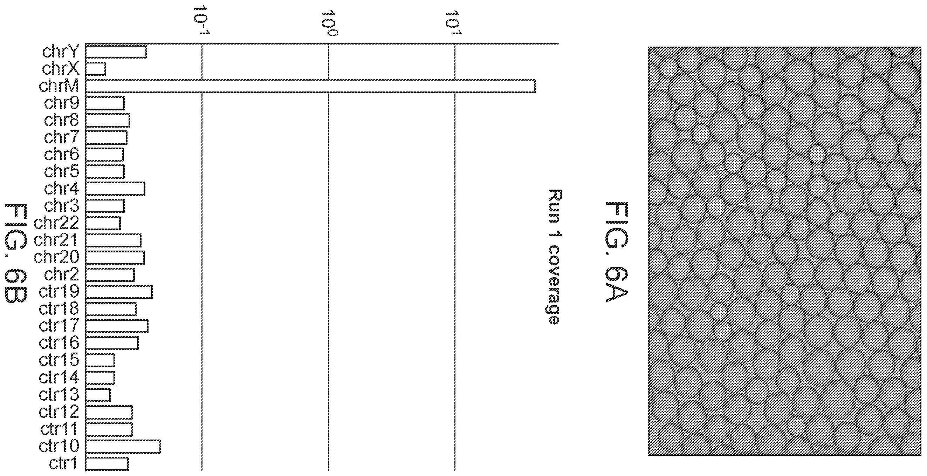

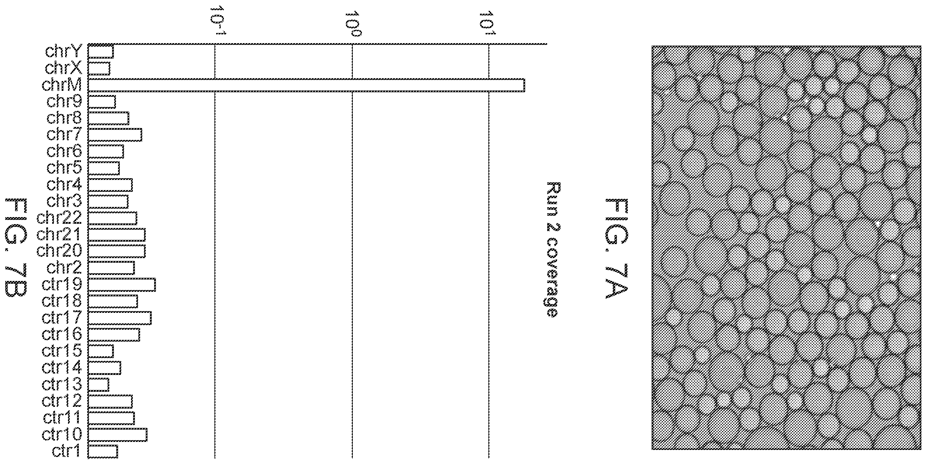

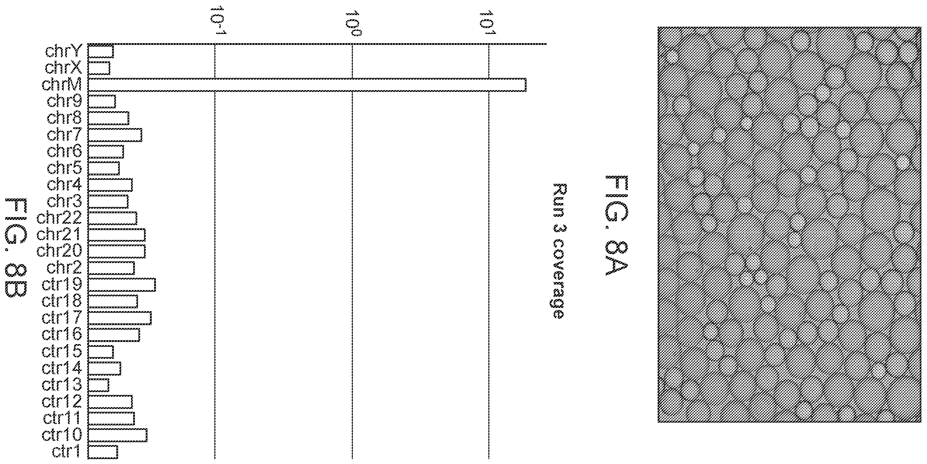

FIG. 6A depicts a 10× microscopy image of droplets post-tagmentation and amplification according to a first experimental run (e.g., Run 1) in which tagmentation is performed in the second droplet. FIG. 7A depicts a 10× microscopy image of droplets post-tagmentation and amplification according to a second experimental run (e.g., Run 2) in which tagmentation is performed in the second droplet. FIG. 8A depicts a 10× microscopy image of droplets post-tagmentation and amplification according to a third experimental run (e.g., Run 3) in which tagmentation is performed in the second droplet. As shown in each of FIGs. 6A, 7A, and 8A, the droplets remained intact and generally monodisperse following tagmentation and amplification. This shows that the tagmentation protocol does not disrupt the droplets and therefore, individual cell lysates are retained within individual droplets.

FIG. 6B depicts normalized coverage across the whole genome according to a first experimental run (e.g., Run 1) in which tagmentation is performed in the second droplet. FIG. 7B depicts normalized coverage across the whole genome according to a second experimental run (e.g., Run 2) in which tagmentation is performed in the second droplet. FIG. 8B depicts normalized coverage across the whole genome according to a third experimental run (e.g., Run 3) in which tagmentation is performed in the second droplet. As shown in each of FIGs. 6B, 7B, and 8B, sequence reads are aligned across the whole genome (e.g., across all 23 autosomal chromosomes, sex chromosomes (chromosome X and Y), as well as mitochondrial DNA).

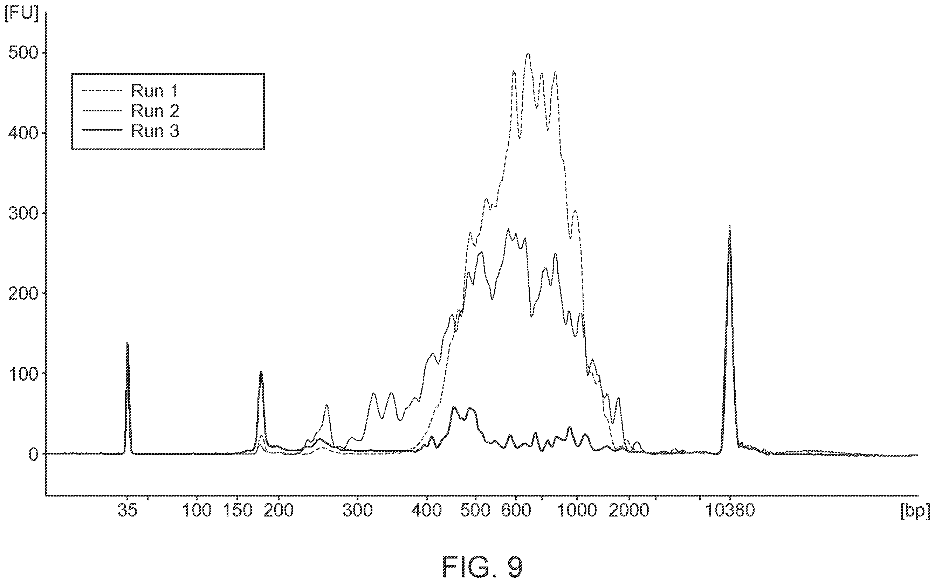

FIG. 9 depicts whole genome library products generated across the first, second, and third experimental runs in which tagmentation is performed in the second droplet. Amplicons of interest are generally found between 150 and 2000 bp in size. Here, amplicons of interest are observed across all three runs. Table 4 shown below further documents the library metrics across the three runs.

**Table 4: Library metrics across runs 1, 2, and 3**

| **Metrics** | **Run 1** | **Run 2** | **Run 3** |
|---|---|---|---|
| % too short | 5.31% | 1.45% | 0.73% |
| % correct structure | 80.49% | 60.01% | 7.79% |
| % mapped | 73.18% | 38.97% | 50.96% |
| Median insert (bp) | 304 | 266 | 236 |

### Example 2: Additional Example of Single Cell Analysis Including Tagmentation in Second Droplet

Mouse (TIB-18) and human (K562) cell lines were mixed (50:50 mix) and processed through the single cell workflow (e.g., Tapestri^{™}) described above in reference to FIG. 1B. In this example, cell lysis and release of genomic DNA occurred in the first droplet followed by tagmentation in the second droplet. Reagents included cell lysis buffer and proteinase K for releasing genomic DNA. The cell lysis protocol in the first droplet included: 1) 50°C for 1 hour, 2) 80°C for 10 minutes, and 3) hold at 4°C. Cell barcodes were additionally added to the second droplet for incorporation into amplicons during nucleic acid amplification.

Two different runs were performed with differing reaction mixture for performing tagmentation. These runs are referred to herein as "Run 4" and "Run 5." The components of the reaction mixture and corresponding volumes for each of the two runs are shown below in Tables 5 and 6. The reaction mixture in the two runs included a ted-WGA enzyme mix which further includes a Tn5 transposase for cleaving and inserting adapters into genomic DNA.

In particular, the reaction mixture of run 4 included a Q5 Master Mix including Q5 High Fidelity DNA polymerase. The reaction mixture of run 5 included a spike in of the Q5 HotStart DNA polymerase. The reaction mixture of run 3 included a spike in of Bst 3.0 DNA polymerase, which is an in silico designed homologue of *Bacillus stearothermophilus* DNA Polymerase I.

**Table 5: Volume of components in reaction mixture for run 4.**

| **Volume** | **Reaction Mixture** |
|---|---|
| 30 uL | ted-WGA buffer mix |
| 30 uL | ted-CapALL enzyme mix - enzyme mix 2 |
| 90 uL | 2X Q5 MM |

**Table 6: Volume of components in reaction mixture for run 5.**

| **Volume** | **Reaction Mixture** |
|---|---|
| 30 uL | ted-WGA buffer mix |
| 30 uL | ted-CapALL enzyme mix - enzyme mix 2 |
| 6 uL | Bst 3.0 |
| 84 uL | dH2O |

For each of runs 4 and 5, droplets including the reaction mixture and cell lysate were processed according to the following preamplification cycling protocol: 1) 1 cycle of 37°C for 30 minutes, 2) 1 cycle of 65°C for 5 minutes, and 3) 1 cycle of 72°C for 3 minutes. Here, the preamplification cycling protocol enables tagmentation and extension to occur. Droplets were exposed to UV for 8 minutes.

For run 4, the subsequent nucleic amplification cycling protocol is as follows: 1) 1 cycle of 98°C for 30 seconds, 2) 10 cycles of 98°C for 10 seconds followed by 72°C for 45 seconds, 3) 10 cycles of 98°C for 30 seconds followed by 61°C for 30 seconds, followed by 72°C for 45 seconds, 4) 1 cycle of 72°C for 3 minutes, and 5) hold at 4°C. For run 5, the subsequent isothermal nucleic amplification cycling protocol is as follows: 1) 1 cycle of 65°C for 2 hours and 2) 80°C for 30 minutes.

Following amplification, amplicons were collected, sequenced, aligned, and distinguished according to presence of cell barcodes.

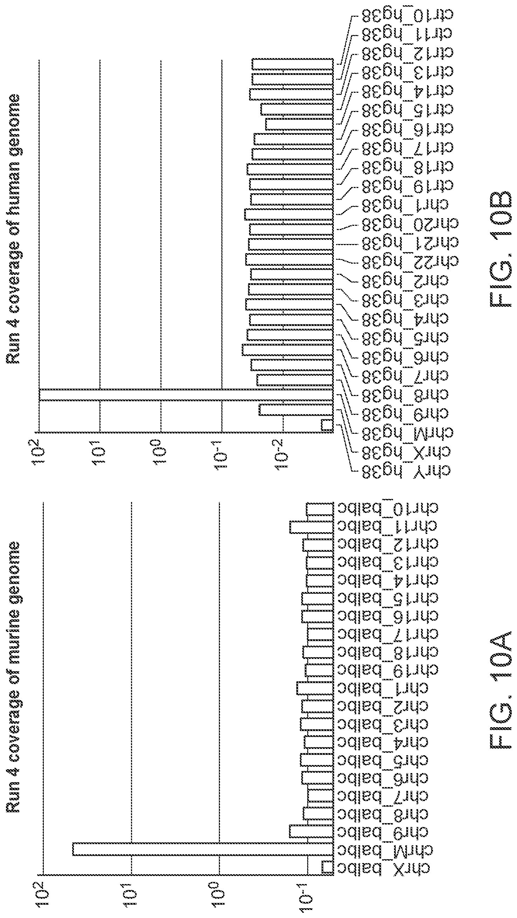

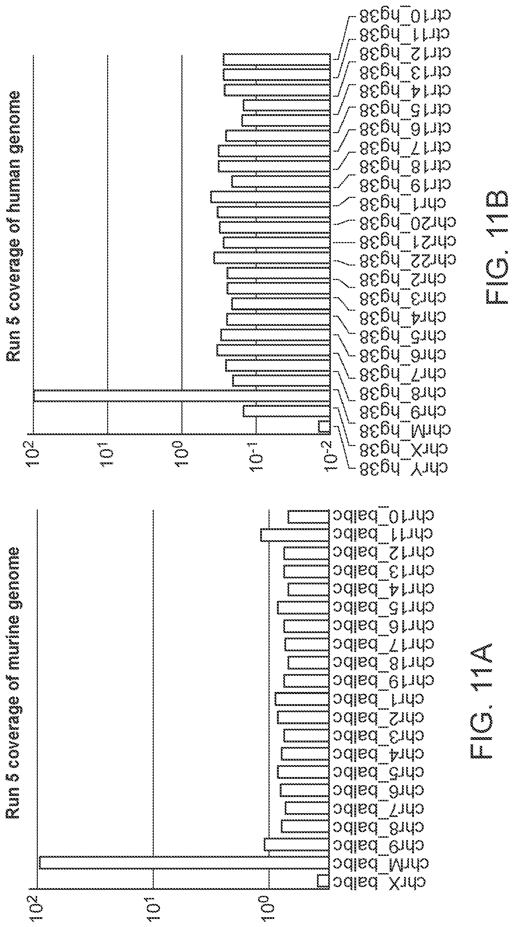

FIG. 10A and FIG. 10B depict normalized coverage across the whole genome of murine (balbc) and human (hg38) cell lines according to a fourth experimental run (e.g., Run 4) in which tagmentation is performed in the second droplet. FIG. 11A and FIG. 11B depict normalized coverage across the whole genome of murine (balbc) and human (hg38) cell lines according to a fifth experimental run (e.g., Run 5) in which tagmentation is performed in the second droplet.

As shown in each of FIGs. 10A and 11A, sequence reads are aligned across the whole mouse genome (e.g., across all 19 autosomal chromosomes, sex chromosomes (chromosome X as cells from only female mice were tested), as well as mitochondrial DNA). Additionally, as shown in each of FIGs. 10B and 11B, sequence reads are aligned across the whole human genome (e.g., across all 22 autosomal chromosomes, sex chromosomes (chromosome X and Y), as well as mitochondrial DNA).

Table 7 shown below further documents the library metrics across the two runs.

**Table 7: Library metrics across runs 4 and 5.**

| **Metrics** | **Run 4** | **Run 5** |
|---|---|---|
| Total Reads | 10,142,221 | 2,536,635 |
| Too short (%) | 10.8% | 26.8% |
| Correct structure (%) | 3.41% | 22.6% |
| Mapped (%) | 25.01% | 18.49% |
| Duplicates (%) | 66.0% | 46.1% |
| Estimated Library Size | 51304 | 137125 |
| Median Insert size (bp) | 145 bp | 265 bp |

### Example 3: Additional Example of Single Cell Analysis Including Tagmentation in First Droplet

Mouse (TIB-18) and human (GM24385) cell lines were mixed (50:50 mix) in DPBS and processed through the single cell workflow (e.g., Tapestri^{™}) described above in reference to FIG. 1B. In this example, cell lysis, release of genomic DNA, and tagmentation occurred in the first droplet followed by barcoding and nucleic acid amplification in the second droplet. For two of the runs, reagents included in the first droplet included cell lysis buffer, protease for releasing genomic DNA, and tagmentation mix (e.g., including transposase). For one of the runs, protease was not included.

Three different runs were performed with differing reagents for performing tagmentation. These runs are referred to in this Example as "Run 6," "Run 7," and "Run 8." The components of the reagents and corresponding volumes for each of the three runs are shown below in Tables 8, 9, and 10. The reagents in the three runs included a ted-CapALL enzyme mix which further includes a Tn5 transposase for cleaving and inserting adapters into genomic DNA. Additionally, the ted-CapALL primer mix further included reverse transcriptase for performing extension after insertion of adapters.

In particular, the reagents of run 6 does not include a protease. Here, run 6 is modeled off of example conventional single-cell workflow processes that do not use proteases. The reagents of run 7 included a proteinase K (Roche PK). The reagents of run 8 included a prepGEM protease.

**Table 8: Volume of components in reagents for run 6 (no protease)**

| **Volume** | **Reagent** |
|---|---|
| 20 uL | ted-CapALL enzyme mix - custom kit |
| 20 uL | ted-CapALL primer mix - custom kit |
| 10.00 uL | NP40 (10%) |
| Up to 100 uL | dH2O |

**Table 9: Volume of components in reagents for run 7 (proteinase K)**

| **Volume** | **Reagent** |
|---|---|
| 0.2165 uL | Roche PK |
| 20 uL | ted-CapALL enzyme mix - custom kit |
| 20 uL | ted-CapALL primer mix - custom kit |
| 10.00 uL | NP40 (10%) |
| Up to 100 uL | dH2O |

**Table 10: Volume of components in reagents for run 8 (prepGEM protease)**

| **Volume** | **Reagent** |
|---|---|
| 1 uL | prepGEM protease |
| 20 uL | ted-CapALL enzyme mix - custom kit |
| 20 uL | ted-CapALL primer mix - custom kit |
| 10.00 uL | NP40 (10%) |
| Up to 100 uL | dH2O |

For Run 6, droplets including the reagents and encapsulated cell were processed according to the following protocol: 1) 1 cycle of 37°C for 30 minutes, 2) 1 cycle of 65°C for 5 minutes, 3) 1 cycle of 72°C for 10 minutes, and 4) hold at 4°C. Here, the protocol enables tagmentation and extension to occur. Given that no protease was included in Run 1, no temperature changes were included for protease activation/inactivation.

For Run 7, droplets including the reagents and encapsulated cell were processed according to the following protocol: 1) 1 cycle of 37°C for 30 minutes, 2) 1 cycle of 65°C for 5 minutes, 3) 1 cycle of 75°C for 60 minutes, 4) 1 cycle of 95°C for 20 minutes, and 4) hold at 4°C.

For Run 8, droplets including the reagents and encapsulated cell were processed according to the following protocol: 1) 1 cycle of 37°C for 30 minutes, 2) 1 cycle of 50°C for 60 minutes, 3) 1 cycle of 80°C for 10 minutes, and 4) hold at 4°C.

For each of Runs 6, 7, and 8, tagmented genomic DNA from individual cells were encapsulated in a second droplet with reaction mixture for performing nucleic acid amplification and cell barcodes. Thus, subsequent nucleic acid amplification generated amplicons that have incorporated the barcodes. Following amplification, amplicons were collected, sequenced, aligned, and distinguished according to presence of cell barcodes.

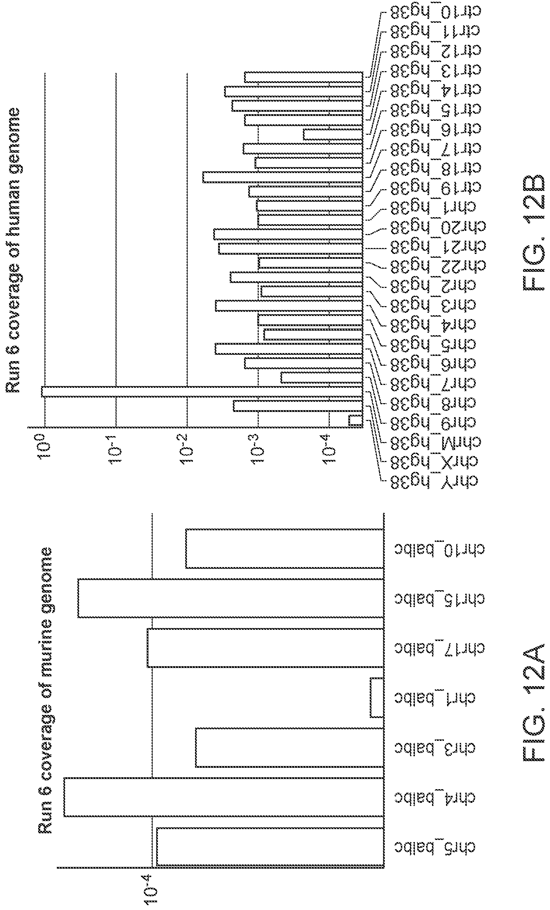

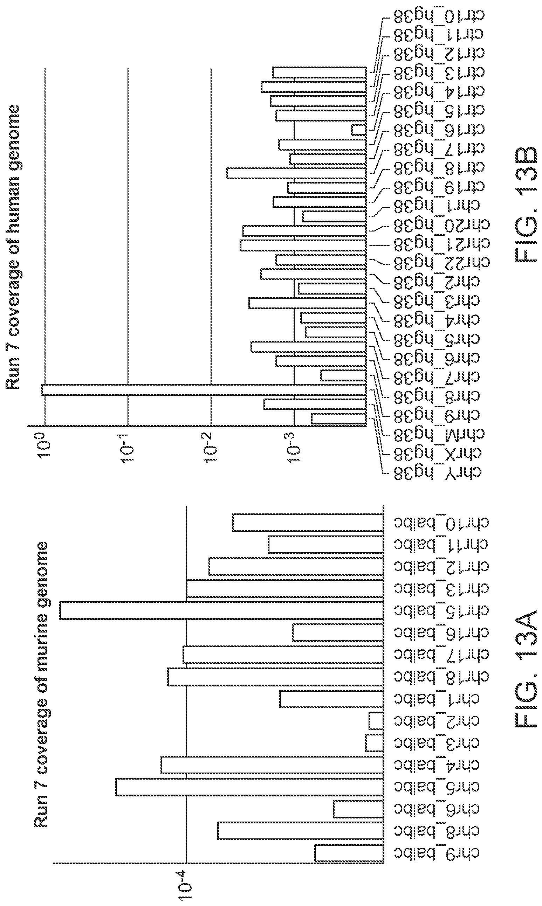

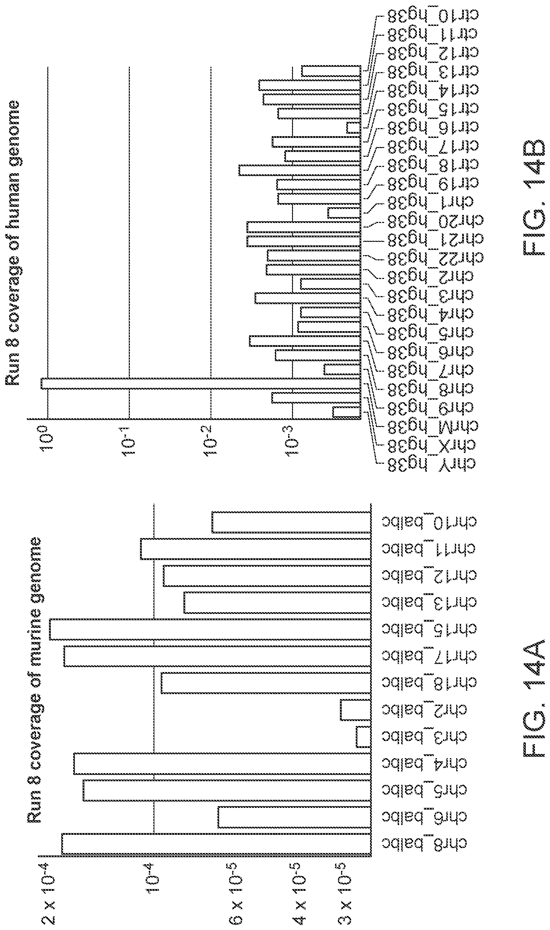

FIG. 12A and FIG. 12B depict normalized coverage across the whole genome of murine and human cell lines according to a sixth experimental run (e.g., Run 6) in which tagmentation is performed in the first droplet. FIG. 13A and FIG. 13B depict normalized coverage across the whole genome of murine and human cell lines according to a seventh experimental run (e.g., Run 7) in which tagmentation is performed in the first droplet. FIG. 14A and FIG. 14B depict normalized coverage across the whole genome of murine and human cell lines according to an eighth experimental run (e.g., Run 8) in which tagmentation is performed in the first droplet.

Generally, run 7 and run 8 (FIGs. 13A/13B and 14A/14B), which included a protease for releasing genomic DNA, achieved improved coverage across the whole genome in comparison to run 6 (FIG. 12A/12B) which did not include a protease. In particular, as shown in FIG. 12A, run 6 achieved limited coverage across the whole genome of mice (e.g., sequence reads only from 7 chromosomes) whereas run 7 achieved coverage across 16 chromosomes and run 8 achieved coverage across 13 chromosomes. This demonstrates the improved access to DNA across the whole genome by incorporating protease in the workflow.

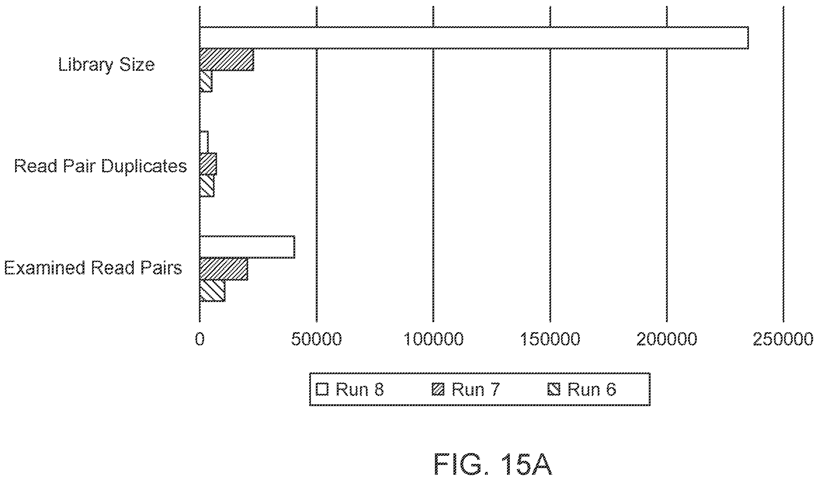

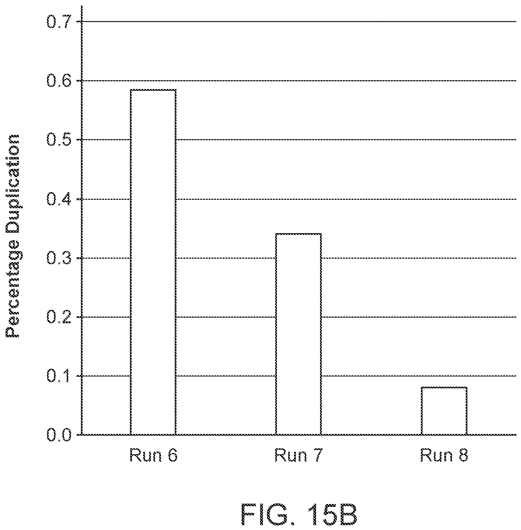

FIG. 15A and FIG. 15B depict library metrics across the sixth, seventh, and eighth experimental runs in which tagmentation is performed in the first droplet. Here, the library metrics shown in FIG. 15A and 15B are measures of library complexity (e.g., library size, duplicates, and read pairs examined). Run 6, which did not include a protease, exhibited the lowest library complexity. Specifically, as shown in FIG. 15A, across the three runs, run 6 achieved the lowest library size and lowest number of read pairs examined. Additionally, run 6 had the second highest number of duplicates. FIG. 15B shows the read duplicates in percentages. Here, run 6 had the higher percentage of duplicate reads, likely due to the lack of access to DNA across the whole genome due to the lack of protease. In contrast, as shown in FIGs. 15A and 15B, run 8 achieved the highest library size, most read pairs examined, and the lowest number and percentage of read duplicates.

Table 11 shown below further documents the library metrics across the three runs. Consistent with the results described above in FIGs. 15A and 15B, Run 6 exhibited the lowest library performance (e.g., highest % of reads that are too short, lowest % of reads with correct structure, lowest % mapped reads) in comparison to Run 7 and Run 8, thereby indicating the value of incorporating a protease into the workflow.

**Table 11: Library metrics across runs 6, 7, and 8.**

| Metrics | No protease (Run 6) | Protease 1 (Run 7) | Protease 2 (Run 8) |
|---|---|---|---|
| Too short (%) | 1.3% | 0.9% | 0.1% |
| Correct structure (%) | 1.1% | 1.5% | 2.0% |
| Mapped (%) | 4.24% | 20.04% | 12.1% |
| Median insert size (bp) | 248 | 251 | 246 |

### Example 4: Metrics of Single Cell Analysis Involving Tagmentation in Either First or Second Droplet

Altogether, Examples 1-3 described above demonstrate that tagmentation can be performed in either a first droplet (along with reagents for cell lysis and genomic DNA release) or a second droplet (along with reaction mixture for barcoding and nucleic acid amplification).

Table 12 below shows the library metrics across performed runs that tagmented genomic DNA in the 1^{st} droplet or the 2^{nd} droplet. Both methods achieved over 3 million total reads, at least 20% of which were successfully mapped. Notably, performing tagmentation in the 2^{nd} droplet resulted in a higher percentage of correct structure reads. Performing tagmentation in the 1^{st} droplet resulted in a higher library complexity (e.g., lower duplicate percentage).

**Table 12: Library metrics comparing tagmenting in the 2^{nd} droplet or in the 1^{st} droplet.**

| Metrics | Tagmentation in 2^{nd} droplet | Tagmentation in 1^{st} droplet |
|---|---|---|
| Total reads | 3,745,782 | 3,762,373 |
| Too short (%) | 3.5% | 0.9% |
| Correct structure (%) | 37.1% | 1.5% |
| Mapped (%) | 24.25% | 20.04% |
| Duplicates (%) | 77.6% | 34.2% |
| Estimated Library size | 19335 | 22676 |
| Median insert size (bp) | 175 | 251 |

## Claims

1. A method for performing whole genome sequencing, the method comprising:
providing a cell and reagents within a first droplet, the reagents comprising a lysing reagent and a protease;
lysing the cell using the lysing reagent within the first droplet;
releasing genomic DNA using the protease within the first droplet by exposing the first droplet to a temperature between 30°C and 60°C;
tagmenting, in either the first droplet or a second droplet, the released genomic DNA by:
using a transposase at a temperature between 35°C and 55°C, cleaving the released genomic and incorporating adaptor sequences into the released genomic DNA; and
filling in one or more gaps in the released genomic DNA created by incorporating the adaptor sequences at a temperature between 40°C and 100°C, optionally using either a reverse transcriptase or DNA polymerase to fill in the one or more gaps; and
amplifying, in the second droplet, the tagmented genomic DNA to generate whole genome amplicons.

2. A method for performing whole genome sequencing, the method comprising:
encapsulating a cell and reagents within a first droplet, the reagents comprising a lysing reagent and a protease;
lysing the cell using the lysing reagent within the first droplet;
releasing genomic DNA using the protease within the first droplet;
encapsulating the released genomic DNA and a reaction mixture in a second droplet, the reaction mixture comprising a transposase and a DNA polymerase;
tagmenting the released genomic DNA in the second droplet by:
using the transposase, cleaving the released genomic and incorporating adaptor sequences into the released genomic DNA;
using the DNA polymerase, filling in one or more gaps in the released genomic DNA created by incorporating the adaptor sequences; and
amplifying, in the second droplet, the tagmented genomic DNA to generate whole genome amplicons.

3. A method for performing whole genome sequencing, the method comprising:
encapsulating a cell and reagents within a first droplet, the reagents comprising a lysing reagent, a protease, and either a reverse transcriptase or DNA polymerase;
lysing the cell using the lysing reagent within the first droplet;
releasing genomic DNA using the protease within the first droplet;
tagmenting the released genomic DNA in the first droplet by:
using the transposase, cleaving the released genomic and incorporating adaptor sequences into the released genomic DNA;
using either the reverse transcriptase or DNA polymerase, filling in one or more gaps in the released genomic DNA created by incorporating the adaptor sequences;
encapsulating the tagmented genomic DNA and a reaction mixture in a second droplet; and
amplifying, in the second droplet, the tagmented genomic DNA using the reaction mixture to generate whole genome amplicons.

4. The method of claim 1 or 3, wherein releasing the genomic DNA and cleaving the released genomic and incorporating adaptor sequences occur in parallel, optionally wherein:
i) releasing the genomic DNA and cleaving the released genomic and incorporating adaptor sequences comprise:
exposing the first droplet to a first temperature between 35°C and 55°C for between 20 minutes and 80 minutes; and
exposing the first droplet to a second temperature between 45°C and 70°C for between 1 minute and 10 minutes; or
ii) releasing the genomic DNA and cleaving the released genomic and incorporating adaptor sequences comprise:
exposing the first droplet to a first temperature of about 37°C for about 30 minutes; and
exposing the first droplet to a second temperature of about 65°C for about 5 minutes.

5. The method of any one of claims 1-4, wherein filling in one or more gaps in the released genomic DNA created by incorporating the adaptor sequences comprises exposing the released genomic DNA to an elevated temperature, optionally wherein:
i) the elevated temperature is at least 40°C, at least 50°C, or at least 60°C; or
ii) filling in one or more gaps uses a reverse transcriptase, and wherein the elevated temperature is between 40°C and 50°C; or
iii) filling in one or more gaps uses a DNA polymerase, and wherein the elevated temperature is between 50°C and 70°C; and/or
iv) wherein the released genomic DNA is exposed to the elevated temperature for between 3 and 8 minutes.

6. The method of claim 5, further comprising exposing the released genomic DNA to a further elevated temperature, optionally wherein the further elevated temperature is at least 70°C, at least 75°C, is between 70°C and 80°C, or is about 72°C.

7. The method of claim 6, wherein:
i) the released genomic DNA is exposed to the further elevated temperature for between 1 minute and 20 minutes, optionally for about 10 minutes; or
ii) wherein the released genomic DNA is exposed to the further elevated temperature for between 40 minutes and 80 minutes, optionally for about 60 minutes.

8. The method of claim 6 or 7, further comprising exposing the released genomic DNA to a yet further elevated temperature, optionally:
i) wherein the yet further elevated temperature is between 90°C and 100°C, or. about 95°C; and/or
ii) wherein the released genomic DNA is exposed to the yet further elevated temperature for between 1 minute and 40 minutes, or for about 20 minutes.

9. The method of any one of claims 1-8, wherein releasing genomic DNA using the protease within the first droplet comprises exposing the first droplet to a temperature between 35°C and 55°C, or about 50°C.

10. The method of any one of claims 1-9, wherein:
i) amplifying the tagmented genomic DNA occurs subsequent to tagmenting the released genomic DNA; and/or
ii) amplifying the tagmented genomic DNA comprises performing one or more cycles of denaturation, annealing, and nucleic acid extension; or performing an isothermal nucleic acid amplification reaction; and/or
iii) amplifying the tagmented genomic DNA using the reaction mixture to generate whole genome amplicons comprises incorporating cell barcodes into the whole genome amplicons

11. The method of any one of claims 1-10, wherein:
i) the lysis reagent is NP40, optionally wherein the lysis agent is 10% NP40; and/or
ii) the protease is proteinase K; and/or
iii) the transposase is a MuA transposase or a Tn5 transposase; and/or
iv) the transposase is a pA-Tn5 fusion transposase; and/or
v) the transposase is appended to the adaptor sequences; and/or
vi) the DNA polymerase is a HotStart DNA polymerase or a *Bacillus stearothermophilus* (Bst) DNA polymerase.

12. The method of any one of claims 1-11, further comprising sequencing the whole genome amplicons.

13. The method of claim 12, further comprising generating a whole genome sequencing library using the sequenced whole genome amplicons, optionally:
i) wherein at least 20%, at least 50%, or at least 80% of sequence reads of the whole genome sequencing library are mapped; or
ii) wherein at least 10%, at least 50%, or at least 80% of sequence reads of the whole genome sequencing library have a correct structure.

14. A system for performing whole genome sequencing according to claims 1-13, the system comprising:
the reagents specified in claims 1, 2 or 3, and a device configured to perform the steps of the method of any of claims 1 to 13;
optionally wherein less than 40% or less than 10% of sequence reads of the whole genome sequencing library are duplicated.

## Patentansprüche

1. Verfahren zum Durchführen einer Gesamtgenomsequenzierung, wobei das Verfahren Folgendes umfasst:
Bereitstellen einer Zelle und von Reagenzien innerhalb eines ersten Tröpfchens, wobei die Reagenzien ein Lysereagenz und eine Protease umfassen;
Lysieren der Zelle unter Verwendung des Lysereagenzes innerhalb des ersten Tröpfchens;
Freisetzen genomischer DNA unter Verwendung der Protease innerhalb des ersten Tröpfchens durch Aussetzen des ersten Tröpfchens gegenüber einer Temperatur zwischen 30 °C und 60 °C;
Tagmentieren der freigesetzten genomischen DNA entweder in dem ersten Tröpfchen oder einem zweiten Tröpfchen durch:
unter Verwendung einer Transposase bei einer Temperatur zwischen 35 °C und 55 °C, Spalten der freigesetzten genomischen DNA und Einbauen von Adaptorsequenzen in die freigesetzte genomische DNA; und
Füllen einer oder mehrerer Lücken in der freigesetzten genomischen DNA, die durch Einbauen der Adaptorsequenzen erzeugt werden, bei einer Temperatur zwischen 40 °C und 100 °C, optional unter Verwendung entweder einer reversen Transkriptase oder DNA-Polymerase, um die eine oder mehreren Lücken zu füllen; und
Amplifizieren der tagmentierten genomischen DNA in dem zweiten Tröpfchen, um Gesamtgenom-Amplicons zu generieren.

2. Verfahren zum Durchführen einer Gesamtgenomsequenzierung, wobei das Verfahren Folgendes umfasst:
Einkapseln einer Zelle und von Reagenzien innerhalb eines ersten Tröpfchens, wobei die Reagenzien ein Lysereagenz und eine Protease umfassen;
Lysieren der Zelle unter Verwendung des Lysereagenzes innerhalb des ersten Tröpfchens;
Freisetzen genomischer DNA unter Verwendung der Protease innerhalb des ersten Tröpfchens;
Einkapseln der freigesetzten genomischen DNA und eines Reaktionsgemischs in einem zweiten Tröpfchen, wobei das Reaktionsgemisch eine Transposase und eine DNA-Polymerase umfasst;
Tagmentieren der freigesetzten genomischen DNA in dem zweiten Tröpfchen durch:
unter Verwendung der Transposase, Spalten der freigesetzten genomischen DNA und Einbauen von Adaptorsequenzen in die freigesetzte genomische DNA;
unter Verwendung der DNA-Polymerase, Füllen einer oder mehrerer Lücken in der freigesetzten genomischen DNA, die durch Einbauen der Adaptorsequenzen erzeugt werden; und
Amplifizieren der tagmentierten genomischen DNA in dem zweiten Tröpfchen, um Gesamtgenom-Amplicons zu generieren.

3. Verfahren zum Durchführen einer Gesamtgenomsequenzierung, wobei das Verfahren Folgendes umfasst:
Einkapseln einer Zelle und von Reagenzien innerhalb eines ersten Tröpfchens, wobei die Reagenzien ein Lysereagenz, eine Protease und entweder eine reverse Transkriptase oder DNA-Polymerase umfassen;
Lysieren der Zelle unter Verwendung des Lysereagenzes innerhalb des ersten Tröpfchens;
Freisetzen genomischer DNA unter Verwendung der Protease innerhalb des ersten Tröpfchens;
Tagmentieren der freigesetzten genomischen DNA in dem ersten Tröpfchen durch:
unter Verwendung der Transposase, Spalten der freigesetzten genomischen DNA und Einbauen von Adaptorsequenzen in die freigesetzte genomische DNA;
unter Verwendung entweder der reversen Transkriptase oder DNA-Polymerase, Füllen einer oder mehrerer Lücken in der freigesetzten genomischen DNA, die durch Einbauen der Adaptorsequenzen erzeugt werden;
Einkapseln der tagmentierten genomischen DNA und eines Reaktionsgemischs in einem zweiten Tröpfchen; und
Amplifizieren der tagmentierten genomischen DNA in dem zweiten Tröpfchen unter Verwendung des Reaktionsgemisches, um Gesamtgenom-Amplicons zu generieren.

4. Verfahren nach Anspruch 1 oder 3, wobei das Freisetzen der genomischen DNA und das Spalten der freigesetzten genomischen DNA und das Einbauen von Adaptorsequenzen parallel erfolgen, optional wobei:
i) das Freisetzen der genomischen DNA und Spalten der freigesetzten genomischen DNA und Einbauen von Adaptorsequenzen Folgendes umfassen:
Aussetzen des ersten Tröpfchens gegenüber einer ersten Temperatur zwischen 35 °C und 55 °C zwischen 20 Minuten und 80 Minuten lang; und
Aussetzen des ersten Tröpfchens gegenüber einer zweiten Temperatur zwischen 45 °C und 70 °C zwischen 1 Minute und 10 Minuten lang; oder
ii) das Freisetzen der genomischen DNA und Spalten der freigesetzten genomischen DNA und Einbauen von Adaptorsequenzen Folgendes umfassen:
Aussetzen des ersten Tröpfchens gegenüber einer ersten Temperatur von etwa 37 °C etwa 30 Minuten lang; und
Aussetzen des ersten Tröpfchens gegenüber einer zweiten Temperatur von etwa 65 °C etwa 5 Minuten lang.

5. Verfahren nach einem der Ansprüche 1-4, wobei das Füllen einer oder mehrerer Lücken in der freigesetzten genomischen DNA, die durch Einbauen der Adaptorsequenzen erzeugt werden, Aussetzen der freigesetzten genomischen DNA gegenüber einer erhöhten Temperatur umfasst, optional wobei:
i) die erhöhte Temperatur mindestens 40 °C, mindestens 50 °C oder mindestens 60 °C beträgt; oder
ii) das Füllen einer oder mehrerer Lücken eine reverse Transkriptase verwendet, und wobei die erhöhte Temperatur zwischen 40 °C und 50 °C liegt; oder
iii) das Füllen einer oder mehrerer Lücken eine DNA-Polymerase verwendet, und wobei die erhöhte Temperatur zwischen 50 °C und 70 °C liegt; und/oder
iv) wobei die freigesetzte genomische DNA zwischen 3 und 8 Minuten lang der erhöhten Temperatur ausgesetzt wird.

6. Verfahren nach Anspruch 5, ferner umfassend Aussetzen der freigesetzten genomischen DNA gegenüber einer weiter erhöhten Temperatur, wobei die weiter erhöhte Temperatur optional mindestens 70 °C, mindestens 75 °C, zwischen 70 °C und 80 °C oder etwa 72 °C beträgt.

7. Verfahren nach Anspruch 6, wobei:
i) die freigesetzte genomische DNA der weiter erhöhten Temperatur zwischen 1 Minute und 20 Minuten lang, optional etwa 10 Minuten lang, ausgesetzt wird; oder
ii) wobei die freigesetzte genomische DNA der weiter erhöhten Temperatur zwischen 40 Minuten und 80 Minuten lang, optional etwa 60 Minuten lang, ausgesetzt wird.

8. Verfahren nach Anspruch 6 oder 7, ferner umfassend Aussetzen der freigesetzten genomischen DNA gegenüber einer noch weiter erhöhten Temperatur, optional:
i) wobei die noch weiter erhöhte Temperatur zwischen 90 °C und 100 °C, oder etwa 95 °C beträgt; und/oder
ii) wobei die freigesetzte genomische DNA der noch weiter erhöhten Temperatur zwischen 1 Minute und 40 Minuten lang oder etwa 20 Minuten lang ausgesetzt wird.

9. Verfahren nach einem der Ansprüche 1-8, wobei das Freisetzen genomischer DNA unter Verwendung der Protease innerhalb des ersten Tröpfchens Aussetzen des ersten Tröpfchens gegenüber einer Temperatur zwischen 35 °C und 55 °C oder etwa 50 °C umfasst.

10. Verfahren nach einem der Ansprüche 1-9, wobei:
i) das Amplifizieren der tagmentierten genomischen DNA nach dem Tagmentieren der freigesetzten genomischen DNA erfolgt; und/oder
ii) das Amplifizieren der tagmentierten genomischen DNA Durchführen eines oder mehrerer Zyklen von Denaturierung, Annealing und Nukleinsäureverlängerung umfasst; oder Durchführen einer isothermen Nukleinsäureamplifikationsreaktion; und/oder
iii) das Amplifizieren der tagmentierten genomischen DNA unter Verwendung des Reaktionsgemisches, um Gesamtgenom-Amplicons zu generieren, Einbauen von Zell-Barcodes in die Gesamtgenom-Amplicons umfasst.

11. Verfahren nach einem der Ansprüche 1-10, wobei:
i) das Lysereagens NP40 ist, optional wobei das Lysemittel 10%iges NP40 ist; und/oder
ii) die Protease Proteinase K ist; und/oder
iii) die Transposase eine MuA-Transposase oder eine Tn5-Transposase ist; und/oder
iv) die Transposase eine pA-Tn5-Fusionstransposase ist; und/oder
v) die Transposase an die Adaptorsequenzen angehängt ist; und/oder
vi) die DNA-Polymerase eine HotStart-DNA-Polymerase oder eine *Bacillus-stearothermophilus(Bst)-DNA-Polymerase* ist.

12. Verfahren nach einem der Ansprüche 1-11, ferner umfassend Sequenzieren des Gesamtgenom-Amplicons.

13. Verfahren nach Anspruch 12, ferner umfassend Generieren einer Gesamtgenomsequenzierungsbibliothek unter Verwendung der sequenzierten Gesamtgenom-Amplicons, optional:
i) wobei mindestens 20 %, mindestens 50 % oder mindestens 80 % der Sequenz-Reads der Gesamtgenomsequenzierungsbibliothek kartiert sind; oder
ii) wobei mindestens 10 %, mindestens 50 % oder mindestens 80 % der Sequenz-Reads der Gesamtgenomsequenzierungsbibliothek eine korrekte Struktur aufweisen.

14. System zum Durchführen einer Gesamtgenomsequenzierung nach den Ansprüchen 1-13, wobei das System Folgendes umfasst:
die in den Ansprüchen 1, 2 oder 3 spezifizierten Reagenzien und eine Vorrichtung, die dazu konfiguriert ist, die Schritte des Verfahrens nach einem der Ansprüche 1 bis 13 durchzuführen;
optional wobei weniger als 40 % oder weniger als 10 % der Sequenz-Reads der Gesamtgenomsequenzierungsbibliothek doppelt sind.

## Revendications

1. Procédé permettant de réaliser un séquençage de génome entier, le procédé comprenant :
la fourniture d'une cellule et de réactifs dans une première gouttelette, les réactifs comprenant un réactif de lyse et une protéase ;
la lyse de la cellule à l'aide du réactif de lyse dans la première gouttelette ;
la libération d'ADN génomique à l'aide de la protéase dans la première gouttelette par l'exposition de la première gouttelette à une température comprise entre 30 °C et 60 °C ;
la tagmentation, dans la première gouttelette ou dans une seconde gouttelette, de l'ADN génomique libéré par :
l'utilisation d'une transposase à une température comprise entre 35 °C et 55 °C, le clivage du génome libéré et l'incorporation de séquences adaptatrices dans l'ADN génomique libéré ; et
le comblement d'une ou de plusieurs brèches dans l'ADN génomique libéré créées par l'incorporation des séquences adaptatrices à une température comprise entre 40 °C et 100 °C, éventuellement l'utilisation d'une transcriptase inverse ou d'une ADN polymérase pour combler la ou les brèches ; et
l'amplification, dans la seconde gouttelette, de l'ADN génomique tagmenté pour générer des amplicons de génome entier.

2. Procédé permettant de réaliser un séquençage de génome entier, le procédé comprenant :
l'encapsulation d'une cellule et de réactifs dans une première gouttelette, les réactifs comprenant un réactif de lyse et une protéase ;
la lyse de la cellule à l'aide du réactif de lyse dans la première gouttelette ;
la libération d'ADN génomique à l'aide de la protéase dans la première gouttelette ;
l'encapsulation de l'ADN génomique libéré et d'un mélange réactionnel dans une seconde gouttelette, le mélange réactionnel comprenant une transposase et une ADN polymérase ;
la tagmentation de l'ADN génomique libéré dans la seconde gouttelette par :
l'utilisation de la transposase, le clivage du génome libéré et l'incorporation de séquences adaptatrices dans l'ADN génomique libéré ;
l'utilisation de l'ADN polymérase, le comblement d'une ou de plusieurs brèches dans l'ADN génomique libéré créées par l'incorporation des séquences adaptatrices ; et
l'amplification, dans la seconde gouttelette, de l'ADN génomique tagmenté pour générer des amplicons de génome entier.

3. Procédé permettant de réaliser un séquençage de génome entier, le procédé comprenant :
l'encapsulation d'une cellule et de réactifs dans une première gouttelette, les réactifs comprenant un réactif de lyse, une protéase et soit une transcriptase inverse soit une ADN polymérase ;
la lyse de la cellule à l'aide du réactif de lyse dans la première gouttelette ;
la libération d'ADN génomique à l'aide de la protéase dans la première gouttelette ;
la tagmentation de l'ADN génomique libéré dans la première gouttelette par :
l'utilisation de la transposase, le clivage du génome libéré et l'incorporation de séquences adaptatrices dans l'ADN génomique libéré ;
l'utilisation de la transcriptase inverse ou de l'ADN polymérase, le comblement d'une ou de plusieurs brèches dans l'ADN génomique libéré créées par l'incorporation des séquences adaptatrices ;
l'encapsulation de l'ADN génomique tagmenté et d'un mélange réactionnel dans une seconde gouttelette ; et
l'amplification, dans la seconde gouttelette, de l'ADN génomique tagmenté à l'aide du mélange réactionnel pour générer des amplicons de génome entier.

4. Procédé de la revendication 1 ou 3, dans lequel la libération de l'ADN génomique et le clivage du génome libéré et l'incorporation de séquences adaptatrices se produisent en parallèle, éventuellement dans lequel :
i) la libération de l'ADN génomique et le clivage du génome libéré et l'incorporation de séquences adaptatrices comprennent :
l'exposition de la première gouttelette à une première température comprise entre 35 °C et 55 °C pendant une durée comprise entre 20 minutes et 80 minutes ; et
l'exposition de la première gouttelette à une seconde température comprise entre 45 °C et 70 °C pendant une durée comprise entre 1 minute et 10 minutes ; ou
ii) la libération de l'ADN génomique et le clivage du génome libéré et l'incorporation de séquences adaptatrices comprennent :
l'exposition de la première gouttelette à une première température d'environ 37 °C pendant environ 30 minutes ; et
l'exposition de la première gouttelette à une seconde température d'environ 65 °C pendant environ 5 minutes.

5. Procédé de l'une quelconque des revendications 1 à 4, dans lequel le comblement d'une ou de plusieurs brèches dans l'ADN génomique libéré créées par l'incorporation des séquences adaptatrices comprend l'exposition de l'ADN génomique libéré à une température élevée, éventuellement dans lequel :
i) la température élevée est d'au moins 40 °C, d'au moins 50 °C ou d'au moins 60 °C ; ou
ii) le comblement d'une ou de plusieurs brèches utilise une transcriptase inverse, et dans lequel la température élevée est comprise entre 40 °C et 50 °C ; ou
iii) le comblement d'une ou de plusieurs brèches utilise une ADN polymérase, et dans lequel la température élevée est comprise entre 50 °C et 70 °C ; et/ou
iv) dans lequel l'ADN génomique libéré est exposé à la température élevée pendant une durée comprise entre 3 et 8 minutes.

6. Procédé de la revendication 5, comprenant en outre l'exposition de l'ADN génomique libéré à une température plus élevée, éventuellement dans lequel la température plus élevée est d'au moins 70 °C, d'au moins 75 °C, est comprise entre 70 °C et 80 °C, ou est d'environ 72 °C.

7. Procédé de la revendication 6, dans lequel :
i) l'ADN génomique libéré est exposé à la température plus élevée pendant une durée comprise entre 1 minute et 20 minutes, éventuellement pendant environ 10 minutes ; ou
ii) dans lequel l'ADN génomique libéré est exposé à la température plus élevée pendant une durée comprise entre 40 minutes et 80 minutes, éventuellement pendant environ 60 minutes.

8. Procédé de la revendication 6 ou 7, comprenant en outre l'exposition de l'ADN génomique libéré à une température encore plus élevée, éventuellement :
i) dans lequel la température encore plus élevée est comprise entre 90 °C et 100 °C, ou est d'environ 95 °C ; et/ou
ii) dans lequel l'ADN génomique libéré est exposé à la température encore plus élevée pendant une durée comprise entre 1 minute et 40 minutes, ou pendant environ 20 minutes.

9. Procédé de l'une quelconque des revendications 1 à 8, dans lequel la libération d'ADN génomique à l'aide de la protéase dans la première gouttelette comprend l'exposition de la première gouttelette à une température comprise entre 35 °C et 55 °C, ou d'environ 50 °C.

10. Procédé de l'une quelconque des revendications 1 à 9, dans lequel :
i) l'amplification de l'ADN génomique tagmenté se produit après la tagmentation de l'ADN génomique libéré ; et/ou
ii) l'amplification de l'ADN génomique tagmenté comprend la réalisation d'un ou de plusieurs cycles de dénaturation, de recuit et d'extension d'acide nucléique ; ou la réalisation d'une réaction d'amplification d'acide nucléique isotherme ; et/ou
iii) l'amplification de l'ADN génomique tagmenté à l'aide du mélange réactionnel pour générer des amplicons de génome entier comprend l'incorporation de codes à barres cellulaires dans les amplicons de génome entier.

11. Procédé de l'une quelconque des revendications 1 à 10, dans lequel :
i) le réactif de lyse est NP40, éventuellement ledit agent de lyse étant NP40 à 10 % ; et/ou
ii) la protéase est la protéinase K ; et/ou
iii) la transposase est une transposase MuA ou une transposase Tn5 ; et/ou
iv) la transposase est une transposase de fusion pA-Tn5 ; et/ou
v) la transposase est annexée aux séquences adaptatrices ; et/ou
vi) l'ADN polymérase est une ADN polymérase HotStart ou une ADN polymérase de *Bacillus stearothermophilus* (Bst).

12. Procédé de l'une quelconque des revendications 1 à 11, comprenant en outre le séquençage des amplicons de génome entier.

13. Procédé de la revendication 12, comprenant en outre la génération d'une banque de séquençage de génome entier en utilisant les amplicons de génome entier séquencés, éventuellement :
i) dans lequel au moins 20 %, au moins 50 %, ou au moins 80 % des lectures de séquences de la banque de séquençage de génome entier sont cartographiées ; ou
ii) dans lequel au moins 10 %, au moins 50 %, ou au moins 80 % des lectures de séquences de la banque de séquençage de génome entier comportent une structure correcte.

14. Système permettant de réaliser un séquençage de génome entier des revendications 1 à 13, le système comprenant :
les réactifs spécifiés dans les revendications 1, 2 ou 3, et un dispositif conçu pour réaliser les étapes du procédé de l'une quelconque des revendications 1 à 13 ;
éventuellement, dans lequel moins de 40 % ou moins de 10 % des lectures de séquences de la banque de séquençage de génome entier sont dupliquées.
